# EUROPEAN PATENT APPLICATION

(11) **EP 1 908 833 A1**
(43) Date of publication of application: **09.04.2008**
(21) Application number: 06756940.0
(22) Date of filing: 02.06.2006
(51) Int. Cl.: C12N 15/12, A01K 67/027, C12Q 1/68, A61K 31/7105, A61P 43/00

(54) **NOVEL TEMPORAL GENE AND APPLICATION OF THE SAME**

(30) Priority: 02.06.2005 JP 2005162943
(71) Applicant: Hisa, Tomoyuki, Izumisano-shi Osaka 598-0046 (JP); Eriguchi, Masazumi, Tokyo 141-0021 (JP)
(72) Inventor: HISA, Tomoyuki, Izumisano-shi, Osaka 598-0046 (JP)
(74) Representative: Rutherford, Jodie
(86) International application number: PCT/JP2006/311119
(87) International publication number: WO 2006/129803

(57) **Abstract**

It is intended to provide a novel gene encoding a new protein which interacts with BMAL2 protein. Namely, a novel gene comprising any one of the following DNAs (a) to (e): (a) a DNA comprising any one of the base sequences represented by SEQ ID NOS: 1 to 4; (b) a DNA that comprises a base sequence derived from the base sequence of the DNA (a) by deletion, substitution or addition of one to several bases and encodes a protein interacting with BMAL2 protein; (c) a DNA that comprises a base sequence derived from the base sequence of the DNA (a) by deletion, substitution or addition of one to several bases and is hybridizable with the DNA (a) under stringent conditions; (d) a DNA that comprises a base sequence derived from the base sequence of the DNA (a) by deletion, substitution or addition of one to several bases and has a homology of 90% or higher with the DNA (a); and (e) a DNA comprising a base sequence that is complementary to any one of the DNAs (a) to (d).

## Description

### Technical Field

The present invention relates to a novel gene for encoding a new protein to interact with a BMAL2 protein encoded by a Bmal2 gene as a clock gene. Furthermore, the present invention relates to a primer of the gene and a probe, a method using thereof for detecting the novel gene, a marker gene for diagnosing a biological rhythm disturbance, and a biological rhythm disturbance model animal.

### Background Art

It has been known that living organisms act by sensing the time, and that the rhythms are of circadian (one day), circatrigintan (one month), circannual (one year) and the like. Such a biological clock indicates, in general, a circadian rhythm as a vital phenomenon that fluctuates in a daily (24 hours) cycle. A human has a cycle of about 25 hours. For a rat, it is about 24.5 hours, and for a mouse, about 23.5 hours. Though there are some differences among the species, the cycle is approximately 24 hours and considered as being reserved as genes in many species.

For the genes relating to the function of the biological clocks, per (period) gene, tim (timeless) gene, Clk (clock) gene, Bmal (brain and muscle Arnt-like) 1 gene, and cyc gene derived from a fly as a homologue of the Bmal1 gene derived from a mammal are known, for example. Regarding proteins encoded by these genes, for example, it has been known that the partner for a CLOCK protein is a BMAL1 protein, and that these proteins activate transcription of the per gene.

These genes are reserved among various species. For example, the cyc gene has been reserved among the fly and the mouse. Furthermore, several kinds of sub-types have been discovered respectively for the genes of per, tim, cyc (= Bmal1) and clk, and four gene families are formed, for example, per, mPer1 (mouse Per1), mPer2, mPer3, tim, mTim, bmal1, Bmal1, and Bmal2 (see Patent document 1), clk and Clk. Moreover, dbt (double-time) gene family (dbt), cry (cyptochrome) gene family (cry, mCry1, mCry2) and the like have been reported. It is also reported that the Bmal2 gene is identical to the Arnt.

Conventionally, the position of the circadian clock has been studied by a primitive method of destroying various sites of the body. Since the circadian rhythm for action disappears when the suprachiasmatic nucleus is destroyed, this site has been considered as the center of the biological clock. Recently however, it is reported newly that the Bmal2 gene is expressed not only in the brain but in the blood vessel (see Non-patent document 1). According to another report, circadian variations of rPer1 (rat Peroid1) and rPer2 are observed by providing a 50% serum irritation to the fibroblast in vitro (see Non-patent document 2).
Patent document 1: JP 2002-238567 A
Non-patent document 1: Circulation 104, 1746-1748, 2001
Non-patent document 2: Cell 93, 929-937, 1998
Non-patent document 3: JBC 275, 36847-36851, 2000

### Disclosure of Invention

### Problem to be Solved by the Invention

For the purpose of avoiding problems caused by abnormalities in the biological rhythm (such as biological rhythm disturbance), the inventors took notice of the Bmal2 gene exhibiting a peripheral expression. The Bmal2 gene belonging to the Bmal1 family is called also a Clif (cycle-like factor) gene (see Non-patent document 3). This is because, as this gene is the Arnt gene itself, functions of the periphery gene are considered to exist certainly. By clarifying the protein that interacts with the BMAL2 protein encoded by the Bmal2 gene, the molecular mechanism of the biological clock can be clarified further.

Therefore, with the foregoing in mind, it is an object of the present invention to provide a novel clock gene encoding novel protein that interacts with a BMAL2 protein. Another object of the present invention is to provide a primer and a probe that can be used for detecting the gene, a method of gene assay using the primer and the probe, a marker gene for diagnosing a biological rhythm disturbance, and a biological rhythm disturbance model animal.

### Means for Solving Problem

For achieving the above-mentioned objects, the novel clock gene of the present invention is a novel gene comprising any one of the following DNAs (a) to (e):
(a) a DNA comprising any one of the base sequences represented by SEQ ID NOS: 1 to 4;
(b) a DNA that comprises a base sequence derived from the base sequence of the DNA (a) by deletion, substitution or addition of one to several bases and encodes a protein interacting with BMAL2 protein;
(c) a DNA that comprises a base sequence derived from the base sequence of the DNA (a) by deletion, substitution or addition of one to several bases and is hybridizable with the DNA (a) under stringent conditions;
(d) a DNA that comprises a base sequence derived from the base sequence of the DNA (a) by deletion, substitution or addition of one to several bases and has a homology of 90% or higher with the DNA (a); and
(e) a DNA comprising a base sequence that is complementary to any one of the DNAs (a) to (d).

The primer of the present invention is a primer for amplifying a polynucleotide comprising the novel gene of the present invention or the partial sequence, and the polynucleotide comprises a partial sequence of the novel gene of the present invention. The probe of the present invention is a probe for hybridizing with the novel gene of the present invention, and the probe is a polynucleotide comprising the novel gene of the present invention or the partial sequence.

The method of the present invention for analyzing a novel gene includes the following two kinds of assays. That is, the first assay includes a step of performing PCR with respect to a test sample by using the primer of the present invention and a step of investigating the existence of the novel gene depending on the existence of a PCR amplified fragment. The second assay includes a step of incubating a test sample and the probe of the present invention, and a step of investigating the existence of the novel gene by detecting hybridization of the test sample and the probe.

The diagnostic marker gene of the present invention is used for diagnosing a biological rhythm disturbance. The gene is the novel gene of the present invention, and the model animal of the present invention is a biological rhythm disturbance model animal in which the novel gene of the present invention is inactivated or overexpressed.

An antibody of the present invention is a polyclonal antibody or a monoclonal antibody with respect to a protein encoded by the novel gene of the present invention or the partial sequence.

A siRNA of the present invention is a double strand siRNA of 21 bases comprising 19 base pairs and 2-base 3' overhang, wherein the sequences of the 19 base pairs are any of the SEQ ID NOS: 26 to 28 of the Sequence Listing.

### Effects of the Invention

As described below, the inventors have identified the novel gene (Erih gene) of the present invention, which encodes a novel protein that interacts with the BMAL2 protein through a two-hybrid assay. In the present specification, the protein encoded by the novel Erih gene of the present invention is called "novel ERIH protein".

As mentioned above, it has been clarified that the novel ERIH protein encoded by the novel Erih gene of the present invention is associated with the BMAL2 protein on the basis of the affinity. From this fact, it is suggested that the ERIH protein controls the transcriptive activity of the BMAL2 protein through the interaction with the BMAL2 protein. Therefore, by using the novel gene of the present invention, for example, the molecular mechanism of the biological clock relating to the association between the BMAL2 protein and the ERIH protein can be clarified further. In addition to that, by detecting the novel Erih gene of the present invention with use of the primer and the probe of the present invention, for example, a genetic biological rhythm disturbance can be diagnosed. Further, since abnormality in the biological rhythm affects adversely the body, the diagnostic marker gene and the model mouse of the present invention are useful for the diagnosis and the clarification of the mechanism.

### Brief Description of Drawings

[FIG. 1] FIG. 1 includes a table and a graph referring to an example of the present invention and indicating relative values of hErih 1 gene expression levels for various organs of a human.
[FIG. 2] FIG. 2 includes a table and a graph referring to an example of the present invention and indicating relative values of hErih2 gene expression levels for various organs of a human.
[FIG. 3] FIG. 3 includes a table and a graph referring to an example of the present invention and indicating relative values of Bmal2 gene expression levels for various organs of a human.
[FIG. 4] FIG. 4 includes a table and a graph referring to another example of the present invention and indicating relative changes of a hErih1 gene using RNAi in a vascular endothelial cell (HPAEC).
[FIG. 5] FIG. 5 includes a table and a graph referring to another example of the present invention and indicating relative changes of a hErih2 gene using RNAi in a vascular endothelial cell (HPAEC).
[FIG. 6] FIG. 6 includes a table and a graph referring to another example of the present invention and indicating relative changes of a Bmal2 gene using RNAi in a vascular endothelial cell (HPAEC).
[FIG. 7] FIG. 7 includes a table and a graph referring to another example of the present invention and indicating relative changes of a hErih2 gene using RNAi in a bronchial/tracheal epithelial cell (NHBE).
[FIG. 8] FIG. 8 includes a table and a graph referring to another example of the present invention and indicating relative changes of a Bmal2 gene using RNAi in a bronchial/tracheal epithelial cell (NHBE).
[FIG. 9] FIG. 9 includes a table and a graph referring to another example of the present invention and indicating relative changes of a hErih1 gene using RNAi in a renal cortical epithelial cell (HRCE) and a renal epithelial cell (HRE).
[FIG. 10] FIG. 10 includes a table and a graph referring to another example of the present invention and indicating relative changes of a hErih2 gene using RNAi in a renal cortical epithelial cell (HRCE) and a renal epithelial cell (HRE).
[FIG. 11] FIG. 11 includes a table and a graph referring to another example of the present invention and indicating relative changes of a Bmal2 gene using RNAi in a renal cortical epithelial cell (HRCE) and a renal epithelial cell (HRE).
[FIG. 12] FIG. 12 is a graph referring to a still another example of the present invention and indicating changes in hErih genes using RNAi in an ovarian cancer cell strain (KF) sensitive to an anticancer agent and in an ovarian cancer cell strain (KFR) that is isolated from the KF and resistant to an anticancer agent.

### Description of the Invention

As described above, the novel gene of the present invention comprises any one of the DNAs of (a) to (e) below:
(a) a DNA comprising any one of the base sequences represented by SEQ ID NOS: 1 to 4;
(b) a DNA that comprises a base sequence derived from the base sequence of the DNA (a) by deletion, substitution or addition of one to several bases and encodes a protein interacting with BMAL2 protein;
(c) a DNA that comprises a base sequence derived from the base sequence of the DNA (a) by deletion, substitution or addition of one to several bases and is hybridizable with the DNA (a) under stringent conditions;
(d) a DNA that comprises a base sequence derived from the base sequence of the DNA (a) by deletion, substitution or addition of one to several bases and has a homology of 90% or higher with the DNA (a); and
(e) a DNA comprising a base sequence that is complementary to any one of the DNAs (a) to (d).

In the above DNAs (b)-(d), the number of bases that can be deleted, substituted or added is 1 to 6 with respect to 50 bases, for example.

In the above DNA (c), the stringent conditions for hybridization can be the standard conditions in the field, for example. For example, the temperature condition is preferably ±5°C from the Tm value of the base sequence represented by the respective SEQ ID NOS, more preferably ±2°C, and further preferably ±1°C. A specific example of the conditions is: 5 x SSC solution; 10 x Denhardt solution; 100 µg/ml salmon sperm DNA; and, hybridization in a 1% SDS at 65°C; irrigation (twice) in a 0.2 x SSC and 1% SDS at 65°C for 30 minutes; and subsequently irrigation (twice) in a 0.2 x SSC and 0.1% SDS at 65°C for 30 minutes.

The homology in the above DNA (d) is at least 90% for example, preferably at least 95%, and more preferably at least 97.5%.

It is also preferable that the novel gene of the present invention has a base sequence represented by SEQ ID NO: 5. It is also preferable that the novel gene includes a polynucleotide comprising a base sequence that encodes an amino acid represented by SEQ ID NO: 6 or a polynucleotide comprising a base sequence complementary thereto. The amino acid sequence represented by SEQ ID NO: 6 is encoded by the base sequence represented by SEQ ID NO: 5, for example.

The novel gene of the present invention is, for example, a gene derived from a mammal such as a human, a mouse, a rabbit, a dog, a cow and a swine. The genes represented by SEQ ID NOS: 1 and 2 are derived from the human, and the genes represented by SEQ ID NOS: 3 and 4 are derived from the mouse.

Examples of the proteins (ERIH protein) encoded by the novel genes of the present invention include:
Mouse ERIH1 protein: GenBank Accession No. AY957601
Human ERIH1 protein: GenBank Accession No. AY946007
Mouse ERIH2 protein: GenBank Accession No. AY957602
Human ERIH2 protein: GenBank Accession No. AY946008

Next, the primer of the present invention is a primer for amplifying the polynucleotide comprising the novel Erih gene of the present invention or the partial sequence, and the primer comprises the partial sequence of the novel gene of the present invention. It is also possible, with application of the PCR using the primer for example, to detect the existence of the novel hErih gene in the test sample or to obtain genes corresponding to the novel hErih gene of the present invention from various living organisms, particularly from mammals.

The length of the primer is not limited particularly, and can be set to a typical length for a primer, for example, in a range of 10 to 35 bases.

Next, the probe of the present invention is hybridizable with the novel Erih gene of the present invention, and the probe is a polynucleotide comprising either the novel Erih gene of the present invention or the partial sequence. By hybridizing the entire novel Erih genes of the present invention or the partial sequences as the probe with a test sample, the existence of the novel Erih gene of the present invention can be detected. Conditions for the hybridization are the same as mentioned above.

Next, the methods of detecting the novel Erih gene of the present invention include a method of using the primer or the probe of the present invention. The detection method using the primer includes a step of performing PCR by using the primer of the present invention with respect to a test sample, and a step of investigating the existence of the novel Erih gene depending on the existence of a PCR amplified fragment. Meanwhile, the detection method using the probe includes a step of incubating the test sample and the probe of the present invention, and a step of investigating the existence of the novel Erih gene by detecting the hybridization of the test sample and the probe. The assay according to the present invention is characterized in use of the primer and the probe of the present invention, and the specific operation and the condition can correspond to conventionally known PCR methods or any of various hybridization methods.

Next, the diagnostic marker gene of the present invention is used for the diagnosis of the biological rhythm disturbance, and characterized in that the gene is the novel Erih gene of the present invention.

A disease model animal of the present invention is a model animal for a biological rhythm disturbance, and the model animal is either an animal (knockout animal) in which the novel Erih gene of the present invention is inactivated or an animal in which the Erih gene is overexpressed. Though there is no particular limitation on the kind of the animal, the examples include a non-human animal like a mouse (for example, a mammal other than a human). The knockout animal or the like can be prepared by any of the conventionally known methods such as gene-targeting, without any particular limitations.

Next, the antibody of the present invention is a polyclonal antibody or a monoclonal antibody with respect to a protein (ERIH protein) encoded by the novel Erih gene of the present invention or the partial sequence. Examples of the partial sequence include a polypeptide comprising an amino acid sequence represented by SEQ ID NO: 6 and the partial sequence.

There is no substantial limitation on the method of preparing the antibody. For example, the polyclonal antibody and the monoclonal antibody can be prepared by a conventionally known method of producing an antibody by immunization to an animal. There is no particular limitation on the kind of the host animal to be immunized. Mammals other than a human, for example, a rabbit, a rat, a mouse, a goat, a sheep, a horse, a swine, and a guinea pig; and birds such as a chicken, a pigeon, a duck and a quail can be used. Similarly, the method of administering the antibody is not limited particularly, and examples of applicable methods include the intracutaneous administration, the subcutaneous administration, the intraperitoneal administration, the intravenous administration and the intramuscular administration. Preferable methods include the subcutaneous administration, the intraperitoneal administration, and the intravenous administration. A further preferable method is the subcutaneous administration.

For example, for preparing a polyclonal antibody, an antibody (ERIH protein or the peptide) is administered to any of the above-mentioned host animals for immunization. Then, the blood serum, ascites fluid or the like are collected, from which the antibody can be isolated and purified. The monoclonal antibody can be prepared by, for example, fusing a myeloma cell and an antibody-producing cell such as a spleen cell or a lymphoidocyte in the immunized host animal so as to prepare a hybridoma, bleeding the hybridoma, and isolating a hybridoma cell that produces an antibody with specificity.

There is no substantial limitation on the method of purifying the polyclonal antibody or the monoclonal antibody. Purification can be performed by any of conventionally known methods such as the salting-out, the dialysis, the ion-exchange chromatography, the affinity chromatography, and the electrophoresis.

There is no particular limitation on the method of screening the production or the target antibody. Conventionally-known methods such as the radioimmunoassay (RIA) technique and the enzyme immunoassay (EIA) technique can be applied, for example.

Typically, the thus obtained antibody has an immunoglobulin class of IgM or IgG. Also the thus obtained antibody molecules can be used directly as an antibody. Alternatively, an active fragment of the antibody such as Fab, Fab', and F(ab')₂ that can be obtained by enzymegenation can be used as an antibody of the present invention.

Next, the siRNA of the present invention is a double strand siRNA targeting the novel Erih gene of the present invention. Though the sequence of the siRNA of the present invention is not limited particularly as long as the target is the novel Erih gene, for example, it is a double strand siRNA of 21 bases comprising 19 base pairs and 2-base 3' overhang, and the sequences of the 19 base pairs are represented by any of SEQ ID NOS: 26 to 28 of the

### Sequence Listing.

In the present invention, "siRNA" denotes a RNA molecule of a short strand that can mediate RNAi, and in general, a double strand low-molecular RNA comprising 21 to 27 bases.

Examples of the double strand siRNA of the present invention include a double strand siRNA targeting both the Erih1 gene and the Erih2 gene, a double strand siRNA targeting the Erih1 gene, and a double strand siRNA targeting the Erih2 gene. Each of these siRNA is a double strand siRNA comprising 19 base pairs and 2-base 3' overhang. An example of sequences of 19 base pairs excepting the overhang in a double strand siRNA targeting both the Erih1 gene and the Erih2 gene is indicated below. These siRNA can be used alone or two or more kinds of siRNA can be used together. In the sequences below, "T" can be recited as "U".

**[Table 1]**

| No. | Sequence | SEQ ID NO |
|---|---|---|
| No. 2 (116) | AGACCAAATTGCTATTAGA | 26 |
| No. 3 (223) | CCAAGTTGTTCAAGCTCTG | 27 |
| No. 4 (236) | GCTCTGCTTACTCAAAAGG | 28 |

In the present invention, the 3' overhang denotes parts of 2mer of 3' end protruding from both ends of the 19 base pairs when two RNA strands (whose 19 bases are complementary sequences) anneal from the 5' end so as to form the double strand. The sequence of the 2-base 3' overhang is not limited particularly, but the examples include TT (-thymine · thymine), AU (-adenine · uracil), and AG (-adenine · guanine). The overhang parts in the sense strand and the antisense strand of the siRNA can have the same or different base sequences. For example, the overhang of the sense strand can be AG and the overhang of the antisense strand can be AU. Alternatively, the overhang of the sense strand can be AU and the overhang of the antisense strand can be AG.

It should be noted that the sequences of the 2-base 3' overhang are not limited to this example. An arbitrary natural nucleic-acid bases (adenine, guanine, thymine, cytosine, uracil), and natural/artificial known modified bases can be used in a range not imposing any substantial influences on the RNAi effect. In general, a ribonucleotide can be used for the nucleotide at the 3' overhang part, but it is not the sole example. A deoxyribonucleotide, a modified ribonucleotide, and any other known nucleotide analogs can be used in a range not imposing substantial influences on the RNAi effect. Furthermore, as required, the double strand siRNA of the present invention can have a 5' overhang in place of the 3' overhang.

Other embodiments for the double strand siRNA of the present invention include a double strand siRNA where one or several bases are modified, substituted, added or deleted in the sequences represented by SEQ ID NOS: 26 to 28 of the Sequence Listing, and which induces the RNA interference specific to the novel Erih gene. Here, 'several' denotes 2, 3, or 4, for example.

The double strand siRNA of the present invention can be synthesized chemically or enzymatically in vitro; it can be synthesized in vivo, without any particular limitations. Preferably, the double strand siRNA is produced through a chemical synthesis by a conventionally-known method. As for a synthetic double strand siRNA, the concentration can be adjusted in a simple manner, and contamination can be prevented easily, and thus there are advantages from the viewpoint of safety. For example, when producing an overhang double strand siRNA represented by SEQ ID NO: 26, first, an RNA strand prepared by adding an overhang of 2 bases to the 3' end of the sequence represented by SEQ ID NO: 26, and also an RNA strand prepared by adding an overhang of 2 bases to the 3' end of a sequence complementary with the sequence represented by SEQ ID NO: 26 are synthesized chemically respectively. Next, the RNA strands are allowed to anneal under a certain condition so as to obtain an overhang double strand siRNA. It is preferable in a practical use that the double strand siRNA is purified suitably by a conventionally known method as required.

A resistance alleviator of the present invention is a medicine for alleviating the cell's resistance to the anticancer agent, and it is characterized in including the double strand siRNA of the present invention. Even for a case of a cancer cell that exhibits resistance to the anticancer agent, the above-mentioned siRNA of the present invention is useful in alleviating the resistance and improving the sensitivity. For this reason, the resistance alleviator including the siRNA of the present invention can be used also in a medical treatment of cells exhibiting resistance to an anticancer agent for example in order to improve the effect of the anticancer agent.

Though the cells to be applied with the agent are not limited particularly, the examples include ovarian cancer cells and the like. Above all, due to the capability of alleviating the resistance to the anticancer agent, the resistance alleviator of the present invention can be used favorably with respect to cells exhibiting resistance to the anticancer agent. There is no particular limitation on the cells. The cells can be human cells, or can be cells of any mammals other than human beings.

### Example 1

By a yeast two-hybrid assay, a BMAL2 protein is used as a decoy protein (bait) so as to screen a novel protein (prey) that interacts with the BMAL2 protein, thereby determining the gene sequence of the novel gene to be a partner of the Bmal2 gene.

### (1) Screening of novel gene (partial sequence) through yeast two-hybrid

In accordance with the assay of two-hybrid system, the following experiment was carried out. First, from a clone of hBmal2 (Invitrogen Clone ID 3445178) purchased from Invitrogen Corporation, a hBmal2 clone was obtained through a PCR cloning. And, using this hBmal2 clone, a BD fusion vector for expressing a fusion protein of a DNA binding protein (BD) and a hBMAL2 protein was prepared. Three kinds of bait insertion fragments for the vector were prepared. Namely, the sequences encode respectively the 1^{st} to the 56^{th} (bait 1), the 193^{rd} to the 283^{rd} (bait 2), and the 125^{th} to 422^{nd} (bait 3) of the amino acids of the hBMAL2 protein (SEQ ID NO: 7). It has been confirmed that the base sequences of the respective bait insertion fragments correspond to the sequences of Invitrogen Clone ID 3445178, and that the respective baits do not have self-activation abilities so that they are effective for screening. A vector formed by binding cDNA of a human vascular endothelial cell and a GAL4 transcription activation domain (AD) was determined as a selection library vector (AD fusion vector). A transformant of a yeast in which the above-mentioned three kinds of BD fusion vectors were introduced and a transformant of yeast in which the above-mentioned AD fusion vectors were introduced were connected by a filter-mating method, and a positive colony was selected (two-hybrid primary screening). As a result, a positive clone was obtained through a screening using the bait 3.

Plasmid was extracted from the thus obtained colony, and the interaction between the bait and prey due to the third promoter-reporter gene system was checked by using a β-gal assay so that the interaction was confirmed. Similarly, the interaction between a bait for a specificity test and the prey was checked by using the β-gal assay so that the high specificity of the prey was confirmed.

Regarding the gene that encodes the prey obtained in the above-mentioned primary screening, a 5' one-pass sequencing was carried out to find that a fragment of a human novel protein not registered in the GenBank is encoded. The thus determined partial base sequence is represented by SEQ ID NO: 5, and the amino acid sequence encoded by the partial base sequence is represented by SEQ ID NO: 6. A protein specifically interacting with the hBMAL2 protein is called "hERIH protein", and a gene encoding this protein is called "hErih gene".

### (2) Determination of entire sequences of hErih gene encoding hERIH protein

The sequences determined in the above paragraph (1) denote sequences of sites of hERIH protein specifically interacts with hBMAL2 protein, and the sites are for reacting with the hBMAL2 protein. Therefore, further the sequences at the 5' side and the 3' side of the hErih protein were determined. Moreover, the entire base sequences were determined, and according to the base sequences, the entire amino acid sequences of the hERIH protein were determined. In the determination of the base sequences, first, the conventionally known 5'RACE method and 3'RACE method were used. Further, based on the information of the partial base sequences in the above (1), genes analogous with the hErih gene were retrieved by use of the computer. As the result showed that the gene was analogous partially with a mouse hypothetical protein XP#358333/NP#766072, the full-length sequence of the hErih gene was determined based on the result and by using PCR and the sequence. As a result, two kinds of base sequences (SEQ ID NOS: 1 and 2) including the above-mentioned sequence represented by SEQ ID NO: 5 were determined. The relatively short gene represented by SEQ ID NO: 1 was named as hErih1 (human Erih1)(endo1), and the relatively long gene represented by SEQ ID NO: 2 was named as hErih2 (human Erih2)(endo2). In a comparison of the exon-intron structures of these genes, exon 11 and exon 12 of the hErih2 gene did not exist in the hErih1 gene.

### Example 2

Expressions of the hErih1 gene and the hErih2 gene in the respective organs of a human were checked.

First, primers suitable for the realtime PCR represented by SEQ ID NOS: 8 to 13 were prepared regarding the Bmal2 gene (ARANT/Bmal2/Clif NM#020183), the hErih1 gene and the hErih2 gene. By using Clontech total RNA Master Panel II (Clontech), the levels of gene expressions for the respective organs were compared. The kinds of organs were 20 in total: adrenal gland, bone marrow, brain cerebellum, brain (whole), fetal brain, fetal liver, heart, kidney, liver, lung (whole), placenta, prostate, salivary gland, skeletal muscle, testis, thymus, thyroid gland, trachea, uterus, and spinal cord. The standard curve was formed with plasmid, and the template was cDNA1 µl (Total RNA: 50 ng chemical equivalent).

### (Primer)

hErih1 gene
SEQ ID NO: 8 Forward primer: 5'- GGGAAGAACCTCAGGAAAAGG-3'
SEQ ID NO: 9 Reverse primer: 5'- CGATCCCACTGTCCAATGTTC-3'
hErih2 gene
SEQ ID NO: 10 Forward primer: 5'- CAGCCCTGTTGCATGTTCA-3'
SEQ ID NO: 11 Reverse primer: 5'- GCCCTGAGAACTGCCCTTTA-3'
Bmal2 gene
SEQ ID NO: 12 Forward primer: 5'- GTGAGCCTGGAGAAGCATCA-3'
SEQ ID NO: 13 Reverse primer: 5'- CCAGCACCAAGTACTGTTCCA-3'

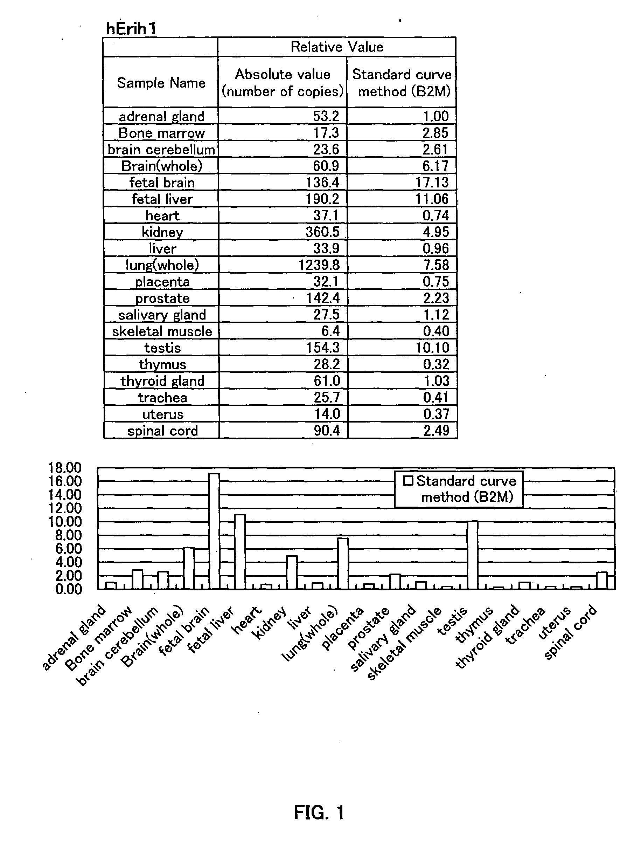

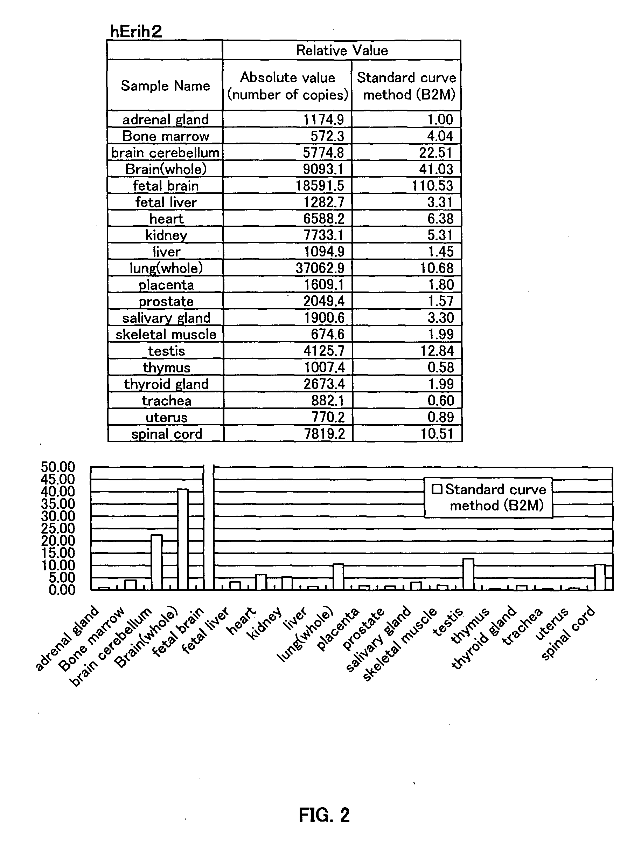

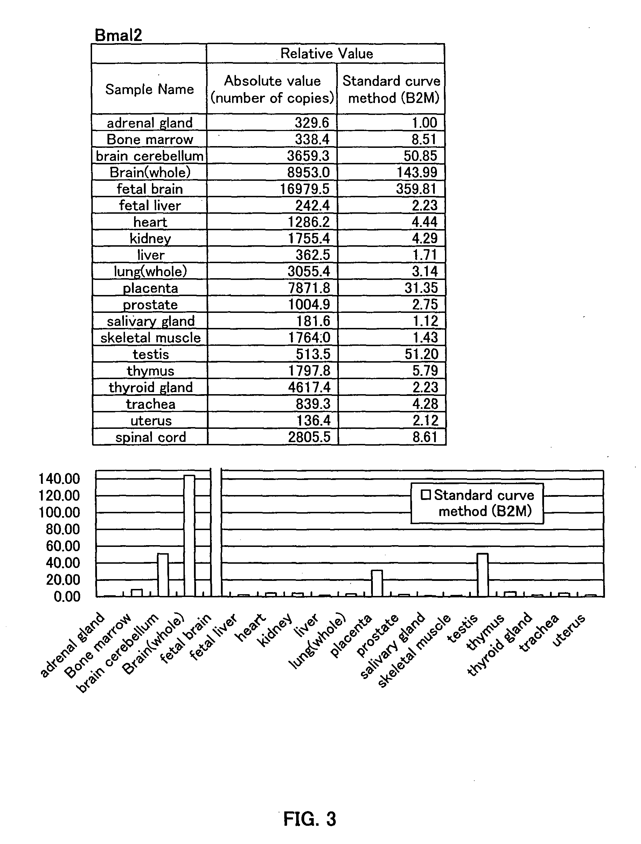

The results are shown in the tables and graphs of FIGs. 1-3. FIG. 1 shows the result for the hErih1 gene, FIG. 2 shows the result for the hErih2 gene, and FIG. 3 shows the result for the Bmal2 gene. The relative values were calculated on the basis of the expression (1.0) of the adrenal gland. As shown in these tables and graphs, a large amount of expression was observed in the brain, the liver and the kidney. For the internal standard gene for expression, Human B2M (beta-2-microglobulin) gene was used. The comparisons in the expression levels after correction are indicated below.

### B2M correction

Bmal2 gene expression: bone marrow < placenta < brain cerebellum < testis < brain (whole) < fetal brain
Erih1 gene expression: kidney < brain (whole) < lung < testis < fetal liver < fetal brain
Erih2 gene expression: bone marrow < lung < testis < brain cerebellum < brain (whole) < fetal brain

### Example 3

Since the clock genes are reserved between different species in general, it was checked whether homologues corresponding to the hErih1 gene and the hErih2 gene exist in animals other than the human.

For the mouse, checking was carried out experimentally by a colony plaque hybridization assay and a PCR assay using a primer designed based on XP#358333/NP#766072. With respect to the animals other than the mouse (i.e., the rat, the rabbit, the dog and the monkey), the analogous retrievals were conducted on the NCBI website.

The result shows that, for the mouse, mErih1 (SEQ ID NO: 3) and mErih2 (SEQ ID NO: 4) corresponding to the hErih1 gene and hErih2 gene were found by the PCR. The result of retrieval of these base sequences by use of a computer showed a partial analogy with NM#172484. As a result of comparison between 'mErih1 gene and mErih2 gene' and 'XP#358333 and /NM#172484', the mErih1 gene and mErih2 gene were regarded as splicing variants. This result implies that the hErih1 gene and hErih2 gene are splicing variants as well.

Concerning the animals other than the mouse, retrieval was conducted on the computer. According to the retrieval result, a gene homologue corresponding to the Erih1 gene and Erih2 gene did not exist for Amphibia, Pisces, and Insecta. In contrast, for Mammalia including the rabbit, the dog, the cow and the swine, the existence of a gene homologue corresponding to the Erih1 gene and Erih2 gene was confirmed.

### Example 4

For the purpose of producing antibodies respectively for a hERIH1 protein, a hERIH2 protein, a mERIH1 protein and a mERIH2 protein, peptides to provide an immunity source were prepared.

For obtaining antibodies that distinguish and specifically identify the respective proteins, parts for distinguishing each gene were cloned to synthesize polypeptides. Specifically, the polypeptides were encoded by the common/specific region of the hErih1 gene and the hErih2 gene, the common/specific region of the mErih1 gene and the mErih2 gene, and the common/specific region of the hErih1 gene and the mErih1 gene. Hereinafter, the respective base sequences, amino acid sequences and the estimated molecular weights are recited.

**[Table 2]**

| Base sequence and amino acid sequence |
|---|
| hErih1 gene-hErih2 gene |
| • Common region: base sequence represented by SEQ ID NO: 14 amino acid sequence represented by SEQ ID NO: 15 |
| • Estimated molecular weight: 21,351.95 Da (196 amino acids) |
| • Specific region: hErih2 gene base sequence represented by SEQ ID NO: 16 amino acid sequence represented by SEQ ID NO: 17 |
| • Estimated molecular weight: 22,440.39 Da (214 amino acids) |
| mErih1 gene-mErih2 gene |
| • Common region: base sequence represented by SEQ ID NO: 18 amino acid sequence represented by SEQ ID NO: 19 |
| • Estimated molecular weight: 31,703.20 Da (286 amino acids) |
| • Specific region: mErih2 gene base sequence represented by SEQ ID NO: 20 amino acid sequence represented by SEQ ID NO: 21 |
| • Estimated molecular weight: 24,898.07 Da (222 amino acids) |
| hErih1 gene-mErih 1 gene |
| • Common region: base sequence represented by SEQ ID NO: 22 amino acid sequence represented by SEQ ID NO: 23 |
| • Estimated molecular weight: 23,201.21 Da (210 amino acids) |
| **•** Specific region: mErih1 gene base sequence represented by SEQ ID NO: 24 amino acid sequence represented by SEQ ID NO: 25 |
| • Estimated molecular weight: 22,379.27 Da (208 amino acids) |

### Example 5

For an analysis of the functions of the hErih genes, an experiment using siRNA was carried out.

The cultivated cells used in this example were derived from the vascular endothelial cell in Example 1 and from the organs (brain, lung and kidney) where more hErih genes were expressed in Example 2. Into the cell culture media, either siRNA regarding the Bmal2 gene or siRNA regarding the hErih gene was added to knock down the Bmal2 gene, the hErih1 gene or the hErih2 gene, and to confirm the changes in the expressions of the Bmal2 gene, the Erih1 gene and the Erih2 gene. At the same time,_changes in the expressions of the other genes were checked by a microarray.

Specifically, HPAEC (pulmonary artery endothelial cell) was used for the vascular endothelial cell; an astrocyte was used for the brain; NHBE (bronchial/tracheal epithelial cell) and SAEC (small airway epithelial cell) were used for the lung; and HRCE (renal cortex epithelial cell) and HRE (renal epithelial cell) were used for the kidney. The standard curves were formed with plasmid, and the template was set to cDNA1 µl (Total RNA 50 ng chemical equivalent). For the internal standard gene for expression, Human B2M (beta-2-microglobulin) gene and Human GAPDH were used. The expression levels after correction were calculated respectively.

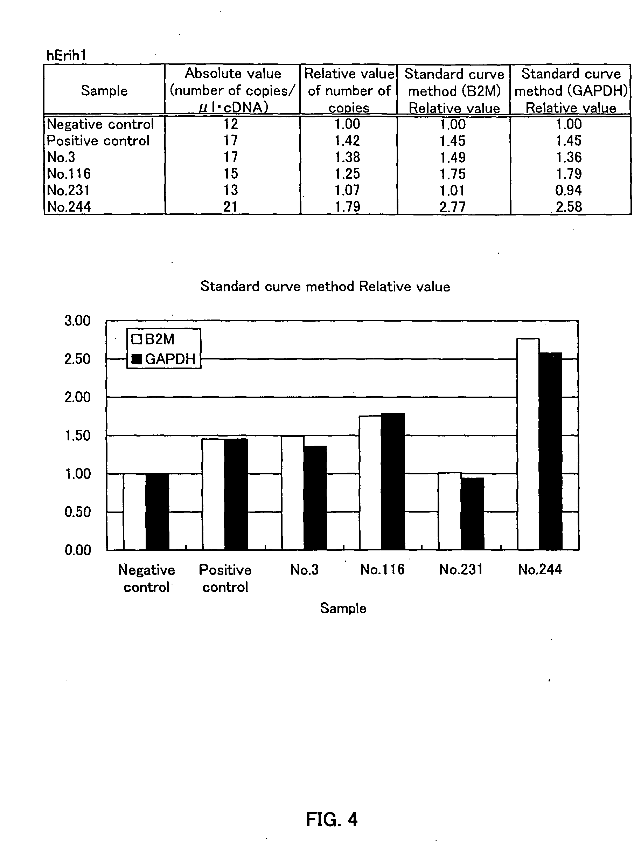

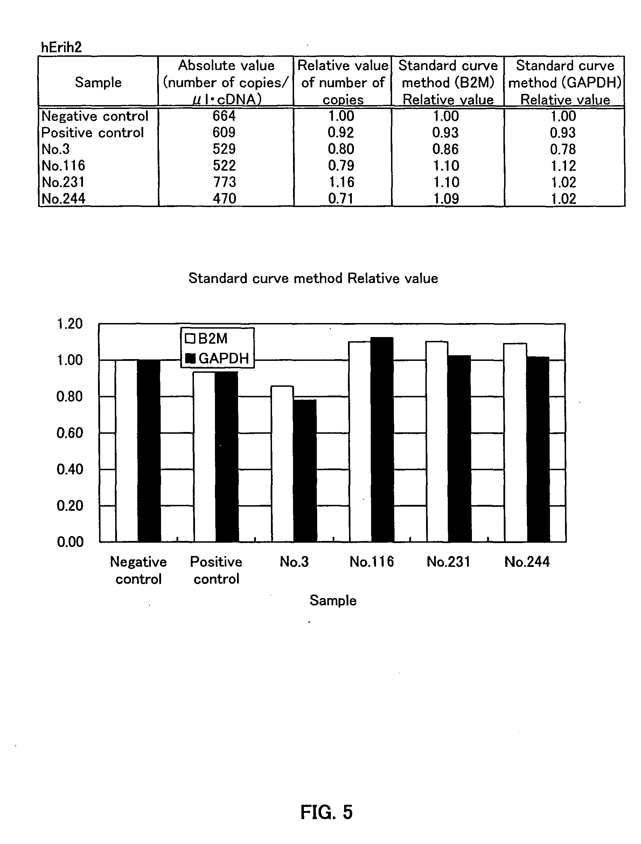

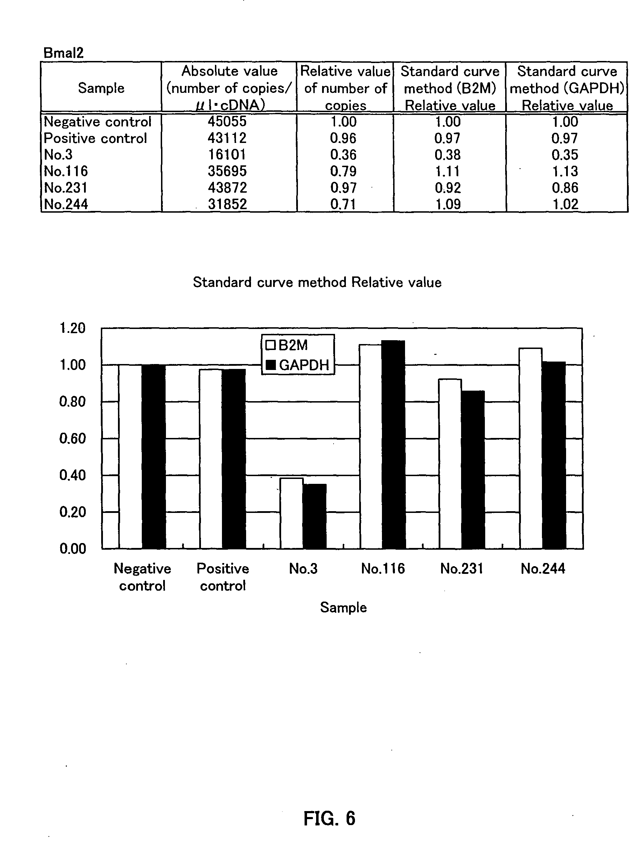

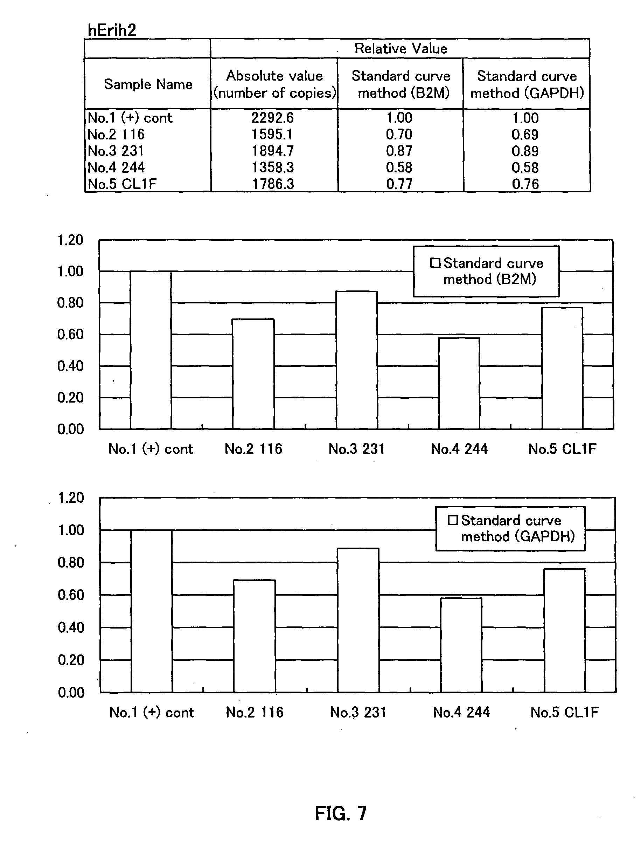

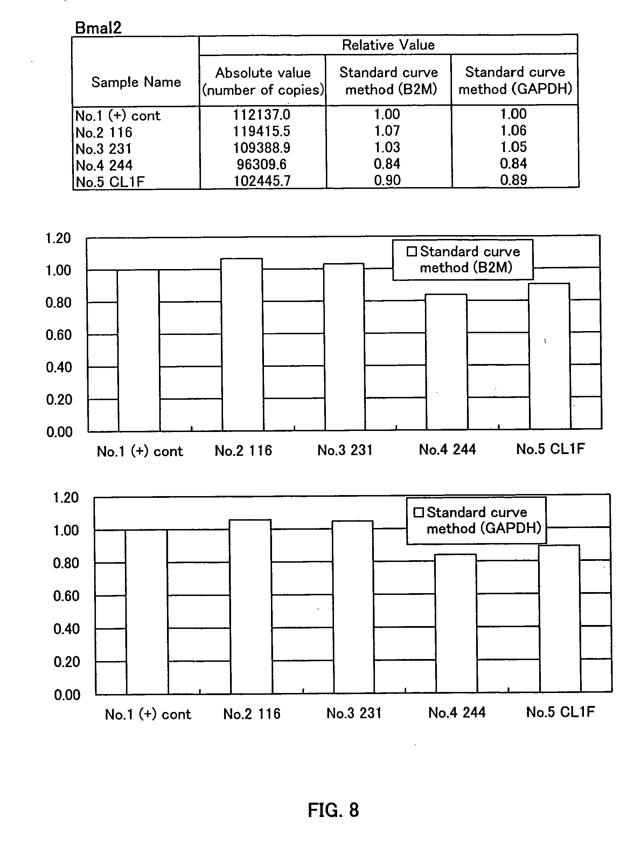

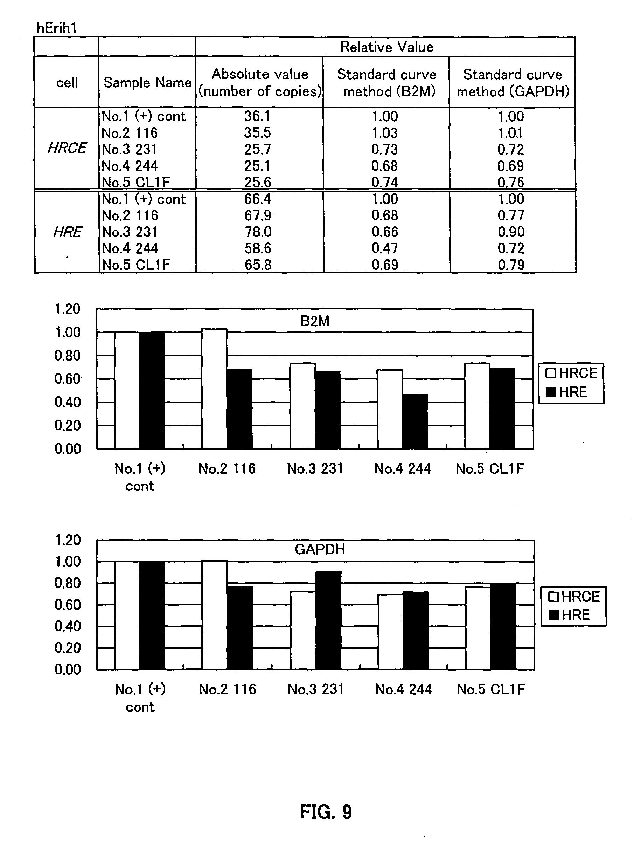

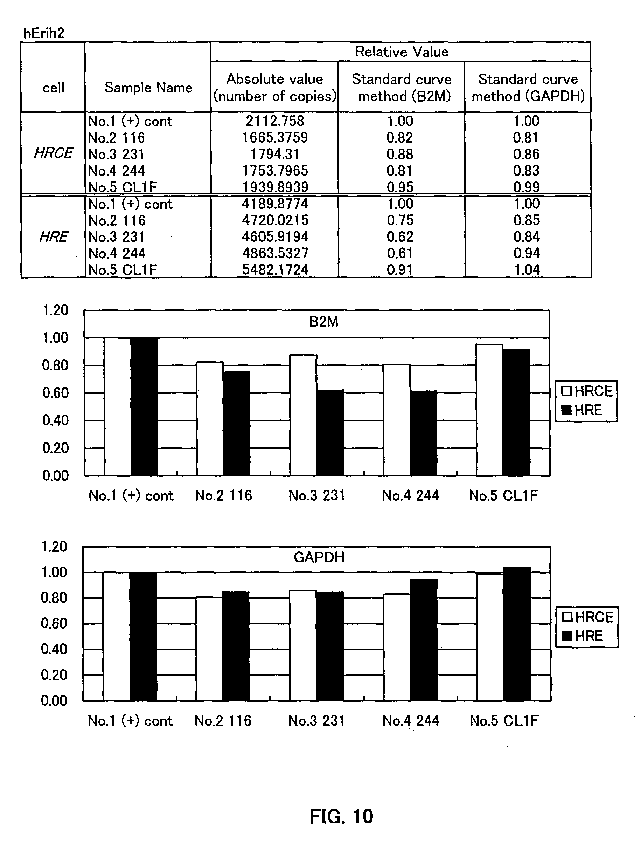

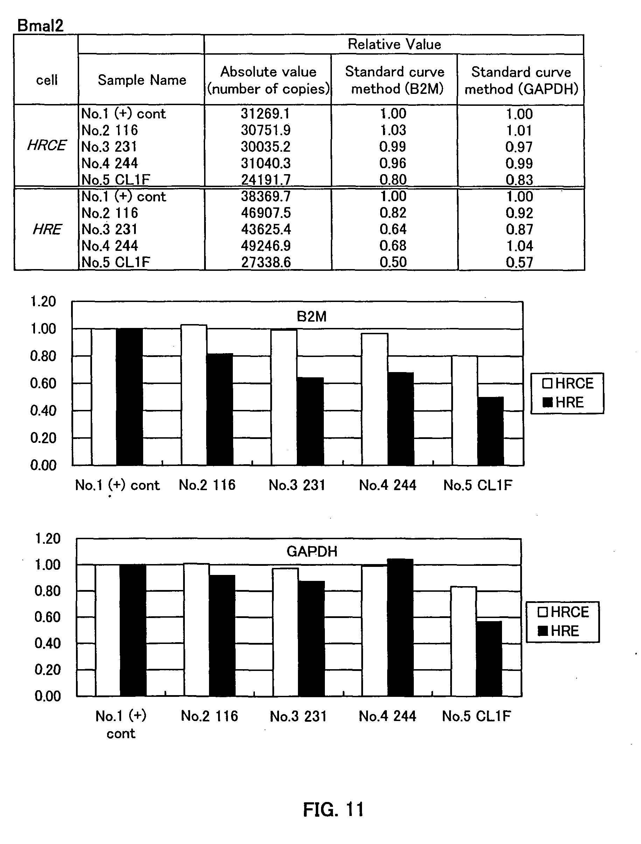

The results for HPAEC are shown in FIGs. 4-6, the results for NHBE are shown in FIGs. 7-8, and the results for HRCE and HRE are shown in FIGs. 9-11 respectively. The controls were not substituted to the knockdown by the siRNA. For the relative values in FIGs. 4-6, the expression of the negative control was set as "1.0" on which the calculation was based. For the relative values in FIGs. 7-11, the expression of the control was set as "1.0", on which the calculation was based.

The sample numbers in the respective drawings are as follows. All of No. 2 (116:116 siRNA), No. 3 (231:231 siRNA) and No. 4 (244: 244 siRNA) are Erih siRNA, which represent the order of the base that was targeted and knocked down, when counted from the 5' side, among the site corresponding to the partial sequence found in Example 1. The sites are first-found corresponding to the partial sequences, and thus the sites are bound specifically with the Bmal2 gene, and also common to both the Erih1 gene and the Erih2 gene. Every siRNA shown in the Table 3 below has a 3' overhang (TT) (in brackets).

**[Table 3]**

| Sample | | SEQ ID NOS and sequence |
|---|---|---|
| No. 1 | Control | |
| No. 116 or No. 2 (116) | siRNA of hErih (#1) | SEQ ID NO: 26 AGACCAAATTGCTATTAGA (TT) |
| No. 3 (223) | siRNA of hErih (#2) | SEQ ID NO: 27 CCAAGTTGTTCAAGCTCTG (TT) |
| No. 4 (236) | siRNA of hErih (#3) | SEQ ID NO: 28 GCTCTGCTTACTCAAAAGC (TT) |
| No. 5 (CLIF) | siRNA of Bmal2 gene (#3) CLIF siRNA=ARNT siRNA = Bmal2 siRNA | SEQ ID NO: 29 CCAGAGTACTGTTGCTGTC (TT) |

Here, each of the siRNAs is a double strand siRNA, and the 3'-end 2 bases (TT) of the sense strand and the antisense strand are overhang (in brackets in the above table).

As shown in these tables, for the Bmal gene, the expression tends to decrease only when Bmal2 siRNA (=Arnt siRNA) is added. In contrast, for the hErin1 gene and the hErih2 gene, the expressions tend to decrease not only in the case of adding Bmal2 siRNA (=Arnt siRNA) but also in the case of adding three kinds of Erih siRNA. Namely, when the Bmal2 gene is suppressed by Bmal2 siRNA, both the Erih1 and Erih2 are suppressed. In contrast, when the Erih1 and Erih2 are suppressed by Erih siRNA, the Bmal2 merely is suppressed. This result suggests that the Bmal2 gene is upstream and the Erih genes are downstream.

### Example 6

In this example, an anticancer-agent-sensitive ovarian cancer cell strain KF and an anticancer-agent-resistant ovarian cancer cell strain KFR isolated from the KF were used to see whether a change to an anticancer-agent sensitivity is observed as a result of addition of siRNA of the Erih gene.

### (Anticancer agent solution)

For the anticancer agent, Oxaliplatin (L-OHP) or Diplatin was used, which was mixed in a solvent so as to prepare an L-OHP solution and a Diplatin solution as anticancer agent solutions.

### (Assay)

A siRNA transfection to cells was carried out using a RNAi Starter kit of QIAGEN and in accordance with its protocol.

First, on the day before the transfection, the cells were suspended in a medium, and 0.5 ml of the suspension was sprayed on 24 well plates and cultivated in a 5% CO₂ atmosphere.

On the day of the transfection, 1 µg of siRNA was diluted to 100 µl in the medium, in which a reagent RNAiFect Transfection Reagent (6 µl) of the above-mentioned kit was mixed. The mixture solution was incubated at room temperature for 10 to 15 minutes in order to form a transfection complex. Meanwhile, the medium was aspirated from the well of the plates cultivating the cells, and a 300 µl of new medium was added as the substitute. The transfection complex (one drop) obtained by the incubation and also the anticancer agent solution were added to the cells in the well, and the plates were rotated gently to homogenize the distribution of the transfection complex and the anticancer agent solution. And the cells were incubated with the transfection complex in the 5% CO₂ atmosphere. The viabilities of the cells were checked in the following manner.

The below-described siRNAs were used alone or in combination with one or two of the other kinds of siRNAs. When two or more kinds of siRNAs were combined, siRNAs of 1 µg in total were diluted in the medium to 100 µl.

**[Table 4] .**

| SiRNA | | SEQ ID NOS and sequence |
|---|---|---|
| B-Mal(2) | siRNA of Bmal2 gene | SEQ ID NO: 29 CCAGAGTACTGTTGCTGTC (TT) |
| Erih(1) | siRNA of hErih (#1) | SEQ ID NO: 26 AGACCAAATTGCTATTAGA (TT) |
| Erih(2) | siRNA of hErih (#2) | SEQ ID NO: 27 CCAAGTTGTTCAAGCTCTG (TT) |
| Erih(3) | siRNA of hErih (#3) | SEQ ID NO: 28 GCTCTGCTTACTCAAAAGC (TT) |

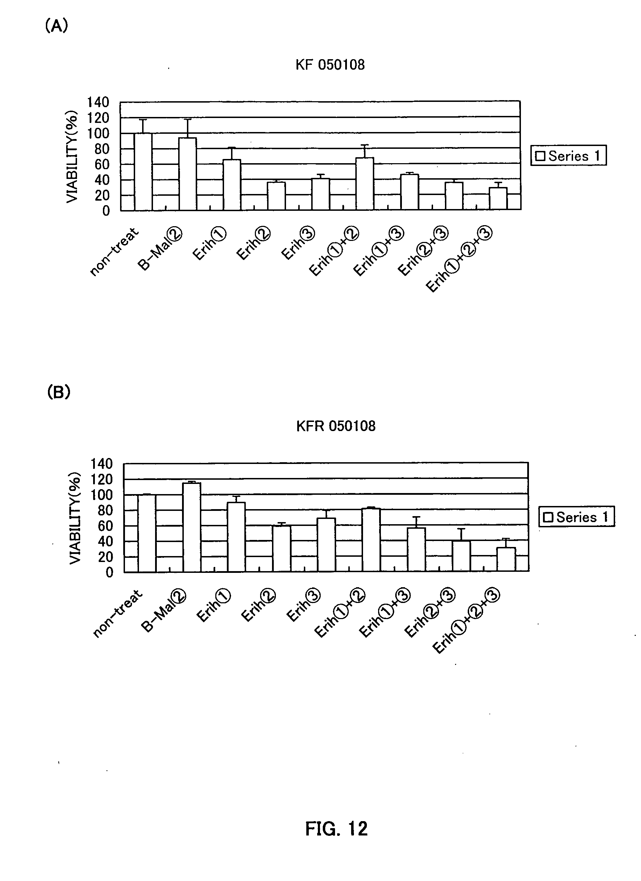

A control (non-treated) was prepared by cultivation in the same manner except that the transfection complex and the anticancer agent solution were not added. The viability (%) was set as a relative percentage when the percentage of the living cells of the control was 100%. The results are shown in FIG. 12. In FIG. 12, the numerical figures in the X-axis denote the numbers of siRNAindicated in the above Table 4. The concentration of the anticancer agent in FIG. 12 denotes the final concentration in the treatment.

FIG. 12 includes graphs for showing the cell viabilities when L-OHP was used as the anticancer agent. FIG. 12(A) shows the result for the sensitive strain KF, and FIG. 12(B) shows the result for the resistant strain KFR. As shown in these graphs, both the anticancer-agent-sensitive strain KF or the anticancer-agent-resistant strain KFR failed to exhibit any significant decrease in the resistance to the anticancer agent. On the contrary, a significant decrease in the resistance to the anticancer agent was observed when RNAi of the Erih gene was added.

### Industrial Applicability

As mentioned above, it has been clarified that the novel Erih gene of the present invention encodes a novel protein that interacts with the BMAL2 protein. Therefore, use of the gene of the present invention and the detection method allow further clarification of the mechanism of the clock gene to which the Bmal2 refers. Furthermore, a diagnosis and an assay of a biological rhythm disturbance can be carried out effectively.

## Claims

1. A novel gene comprising any one of the following DNAs (a) to (e):
(a) a DNA comprising any one of the base sequences represented by SEQ ID NOS: 1 to 4;
(b) a DNA that comprises a base sequence derived from the base sequence of the DNA (a) by deletion, substitution or addition of one to several bases and encodes a protein interacting with BMAL2 protein;
(c) a DNA that comprises a base sequence derived from the base sequence of the DNA (a) by deletion, substitution or addition of one to several bases and is hybridizable with the DNA (a) under stringent conditions;
(d) a DNA that comprises a base sequence derived from the base sequence of the DNA (a) by deletion, substitution or addition of one to several bases and has a homology of 90% or higher with the DNA (a); and
(e) a DNA comprising a base sequence that is complementary to any one of the DNAs (a) to (d).

2. The novel gene according to claim 1, comprising a polynucleotide comprising a base sequence represented by SEQ ID NO: 5.

3. The novel gene according to claim 1, comprising either a polynucleotide comprising a base sequence encoding an amino acid sequence represented by SEQ ID NO: 6 or a polynucleotide comprising a complementary base sequence.

4. The novel gene according to claim 1, wherein the gene is derived from at least one mammal selected from the group consisting of a human, a mouse, a rabbit, a dog, a cow and a swine.

5. A primer for amplifying a polynucleotide comprising the novel gene according to claim 1 or the partial sequence, the primer being a polynucleotide comprising the partial sequence of the novel gene according to claim 1.

6. The primer according to claim 5, the primer being a polynucleotide comprising a base sequence represented by SEQ ID NO: 5 or a polynucleotide comprising the partial sequence.

7. A method of detecting the novel gene according to claim 1, the method comprising steps of: performing PCR with respect to a test sample by using the primer according to claim 5; and investigating the existence of the novel gene in accordance with the existence of a PCR amplified fragment.

8. A probe hybridizable with the novel gene according to claim 1, the probe being a polynucleotide comprising the novel gene according to claim 1 or the partial sequence.

9. A method of detecting the novel gene according to claim 1, comprising steps of: incubating a test sample and the probe according to claim 8; and detecting the hybridization of the test sample and the probe so as to investigate the existence the novel gene.

10. A diagnostic marker gene used for a diagnosis of a biological rhythm disturbance, the gene being the novel gene according to claim 1.

11. A biological rhythm disturbance model animal, wherein the novel gene according to claim 1 is deactivated or overexpressed.

12. A double strand siRNA of 21 bases comprising 19 base pairs and 2-base 3' overhang, wherein the sequences of the 19 base pairs are any of SEQ ID NOS: 26 to 28 of the Sequence Listing, and the target is the novel gene according to claim 1.

13. A resistance alleviator for alleviating a resistance of a cell to an anticancer agent, the alleviator comprising the double strand siRNA according to claim 12.

14. The resistance alleviator according to claim 13, wherein the cell is an ovarian cancer cell.

15. The resistance alleviator according to claim 13, wherein the cell is derived from a mammal.


SEQUENCE LISTING

<110> ERIGUCHI, Masazumi
      HISA, Tomoyuki

<120> Novel Clock Gene And Appliction Of The Same

<130> H2645

<150> JP 05/162943
<151> 2005-06-02

<160> 29

<170> PatentIn version 3.1

<210> 1
<211> 1569
<212> DNA
<213> Homo sapiens

<400> 1
atgcatgaga agctgataca tgaactggaa gaggagagac acttacgtct tcaaagcgag      60

aagcggttgc aggaggtgac cctagagtct gaacgcaaca gaattcagat gcgtagcttg     120

cagcagcagt tctccaggat ggaagaaaca gtacgaaatc tattgcagag tcaaggatct     180

ccagagcaga aaaaagaaga aactgttaat ataatggtct atcaggaaaa gctgtcagag     240

gaagagagaa aacataagga agctttggaa gatcttcaca tggtggttga tgaggattcg     300

aggagtgaaa gcagcagtac agatgaggga aaagaaaaga ccaaattgct attagagaga     360

ttgaaagctc tagaggcaga gaattcagcg ttggctttgg agaatgaaaa tcaaagggaa     420

caatatgagc gatgtcttga tgaggtagcc aaccaagttg ttcaagctct gcttactcaa     480

aaggatctaa gagaggaatg tgtgaagttg aaaacaagag tgtttgattt ggaacagcag     540

aatcgaacac taagcatcct attccaacag cgagtcagac ccacttctga tctgctcctc 600

cagaaacttc actcacgtct cttggatctt tcatctggag atttgctttc agaggtggaa 660

agaaaccgaa gtctgacaca gtcacgaact gatgctgagg tgcacgaaca ccaactaaat 720

acaaaatcgg ccttgaaatg ccctggcttg gggctgtca tccctggtca tctctgtcct 780

cgaaacagct acagtagcag cagtgaactg tctctttcaa gcacctgcag cgagtactcc 840

agtggctcct cctacacgtg gcacgatggg aagaacctca ggaaaagggt aaaagaggca 900

aatgaaaact tgcaagagga tgaagacgat gcagttgcag attctgtatt tcagagccac 960

atcatagaat ccaactgcca gatgagaaca ttggacagtg ggatcggaac ctttccactc 1020

ccagactcgg gaaatcgctc gacaggacgc tacctatgcc agccagactc cccagaggac 1080

gctgagcctc tcctgcctct ccagtcagcc ctttctgcag tttcttccat gagagcccaa 1140

acccttgaac gtgaagtgcc ttcctccaca gacggccagc gccctgcaga tagcgccatt 1200

gttcattcca catccgaccc catcatgacc gccagaggga tgaggcctct tcagagccgc 1260

ctccccaaac cagcttcctc aggaaaagtc agttcccaaa agcagaatga agcagagcca 1320

aggcctcaga catgctcatc attcggatat gctgaagacc caatggcaag ccagccgctt 1380

ccagactggg ggagtgaagt tgctgccacc gggacccagg acaaggcacc cagaatgtgt 1440

acgtactctg ccagcggtgg cagtaatagt gacagtgacc tggactatgg agataatggt 1500

tttggagctg gaaggggaca gttagtgaaa gcactgaaga gcgctgcccc agaaattgag 1560

acaacttga 1569

<210> 2
<211> 5526
<212> DNA

<213> Homo sapiens

<400> 2
atgcatgaga agctgataca tgaactggaa gaggagagac acttacgtct tcaaagcgag 60

aagcggttgc aggaggtgac cctagagtct gaacgcaaca gaattcagat gcgtagcttg 120

cagcagcagt tctccaggat ggaagaaaca gtacgaaatc tattgcagag tcaaggatct 180

ccagagcaga aaaaagaaga aactgttaat ataatggtct atcaggaaaa gctgtcagag 240

gaagagagaa aacataagga agctttggaa gatcttcaca tggtggttga tgaggattcg 300

aggagtgaaa gcagcagtac agatgaggga aaagaaaaga ccaaattgct attagagaga 360

ttgaaagctc tagaggcaga gaattcagcg ttggctttgg agaatgaaaa tcaaagggaa 420

caatatgagc gatgtcttga tgaggtagcc aaccaagttg ttcaagctct gcttactcaa 480

aaggatctaa gagaggaatg tgtgaagttg aaaacaagag tgtttgattt ggaacagcag 540

aatcgaacac taagcatcct attccaacag cgagtcagac ccacttctga tctgctcctc 600

cagaaacttc actcacgtct cttggatctt tcatctggag atttgctttc agaggtggaa 660

agaaaccgaa gtctgacaca gtcacgaact gatgctgagg tgcacgaaca ccaactaaat 720

acaaaatcgg ccttgaaatg ccctggcttg ggggctgtca tccctggtca tctctgtcct 780

cgaaacagct acagtagcag cagtgaactg tctctttcaa gcacctgcag cgagtactcc 840

agtggctcct cctacacgtg gcacgatggg aagaacctca ggaaaaggca atcatcacaa 900

aactgggata aaaggctaag tattgattct tcgctcccaa gtggctttgc tagtcctaca 960

aatgaactac ctccaactcg tatcaaggaa agccacattt tggaagggct aagaaagcta 1020

cagaagcgaa aagtgttact tgaaccccca tcagtgataa ccaaatgggg ttataaagat 1080

tgcatgaact cgaatgaagg aatatattct cctggaatta aaagtagcag cctcaaggag 1140

tatcccccct gcaaaacagc tgacctgggg agcccctgca aggaacccca caagacattt 1200

gtttatgatc tagattccca cgttgatgcg gacgatgacc cttccacctt agcattgctc 1260

caagcagttc caaaccagag ctgcaggcca catggcagta aattaaccca cagtgtttcc 1320

gacagtctgt ttggctggga gacaaacaga aaacactttc tggaaggcac atcctcagtt 1380

tatcccaagg aaaggcctga aaagctgaca agttgcgcca gcagctgtcc cttagagatg 1440

aagctgtgcc caagtgtgca gacgcctcag gtacagaggg agagggcccc acagggccaa 1500

ggccatggcc gcatggctct caacctccag ctttcagaca ctgatgacaa tgaaacgttt 1560

gatgagctgc acatagagag cagtgatgag aaaagtcctt cagacgtgtc attggctgcc 1620

gacaccgata agtccgtgga gaacctggat gtccttgtgg ggtttggaaa atctctatgt 1680

gggtctcctg aagaggagga aaaacaagtg cccatccctt cagagactag gccaaagact 1740

tttagtttca ttaagcagca aagagttgta aaaaggactt cttcagaaga atgtgtgact 1800

gtgatatttg atgcggaaga cggtgagccc attgaattca gctctcacca gactggggtt 1860

gtcactgtta ccagaaatga gatttccatc aattcaactc ctgctggacc caaggcagaa 1920

catactgagc ttttacctca gggaattgct tgtttacagc caagagctgc tgcaagagac 1980

tatactttct ttaaaaggtc tgaagaggac actgagaaaa acattccaaa agataatgta 2040

gataatgttc ccagggtgtc cactgaatct ttcagctcca ggacagtgac acaaaatcct 2100

cagcagcaaa agctggtcaa accaacacac aatatatcat gccagagtaa ttccaggtct 2160

tcggcaccca tgggcatcta tcaaaagcaa aatctgacaa aaatacctcc caggggcaag 2220

tcttcacctc agaaatcaaa actaatggag cccgaagcca ccacactact cccttcatct 2280

ggcctggtga ctcttgaaaa atcaccagcc ttagctcctg ggaaactctc acgattcatg 2340

aagactgaga gctcagggcc cctctttgaa ttacgatcag atccacacat tccaaaacat 2400
tccgcccaac ttccgcacag ctccaggatg cccagcagga gggactgggt ccagtgcccc 2460
aagagtcaga ctccagggtc acggtcaagg cctgccattg agtctagtga cagtggagag 2520
ccccccacga gggatgaaca ctgtggctct gggccggagg caggggtgaa atccccttcc 2580
cctccgcccc ctccaggcag gtccgtctcc ctgctggcca ggcccagcta tgactattca 2640
ccagcacctt catccaccaa gtccgaaacc agggtcccca gtgaaacagc aaggacccca 2700
ttcaaatccc cgctgctgaa aggaatttct gctccagtta tttcttctaa tccggccacg 2760

acagaagtgc agaggaagaa accttctgtg gccttcaaaa agcctatctt cactcaccct 2820
atgccctccc cagaagcagt cattcaaacc cgatgccctg ctcatgcccc ctccagctcc 2880
ttcaccgtaa tggctctggg gcctccaaag gtctctccga agagaggtgt ccccaaaacc 2940
tctcctcgcc aaacacttgg gaccccacaa agggacatag gattacagac tcccaggatc 3000
tctccttcaa cccatgagcc actggaaatg acgtcctcca aaagtgtatc tccagggaga 3060
aaaggacaat tgaatgatag cgcctccaca ccccccaagc cttccttctt aggggtaaat 3120
gagtcaccat catctcaggt cagcagttcc tcatcatcct catcacccgc caaaagccat 3180
aacagccctc atggttgtca aagtgctcat gagaaaggac tgaaaactcg ccttccagtg 3240
ggactcaaag tgctcatgaa gtctccccag ctgctcagga aaagttccac cgtgccaggg 3300
aaacatgaaa aagacagttt aaatgaagcc tccaaaagtt ccgtggctgt gaacaagtct 3360
aagccagagg actccaagaa tccagcaagc atggagatca cagcgggtga aagaaatgtg 3420
accctaccgg attcacaagc acagggcagt ttagctgatg ggcttcccct ggaaacagca 3480

ctacaagagc cattggaaag tagcatccct gggagtgatg gaagggatgg ggtagataat 3540

agatccatga gaagatccct ttcctccagc aaaccacacc taaaaccagc tctgggtatg 3600

aatggcgcca aagcccgcag ccacagcttc agtacacact caggagacaa gccttctacg 3660

ccccccatcg aagggtcagg caaagtccgc actcagatca ttaccaatac cgccgagaga 3720

ggcaattctc ttacccggca gaactcttcc acggaaagct ctcccaacaa ggccccttct 3780

gctcccatgt tggagagtct ccccagtgtt gggaggccct cggggcaccc ctcctccggg 3840

aagggctccc tggggagctc aggcagcttc agcagtcagc atgggagccc aagtaagttg 3900

cctttgagga tccctccaaa gtctgaggga ctcctcatcc cacctggaaa ggaagaccag 3960

caggccttca cccagggaga gtgccccagt gccaatgtgg ctgtacttgg ggaaccaggc 4020

agtgaccgcc gcagttgccc acccacccca acagactgcc ctgaagccct gcagagccca 4080

gggaggactc agcatccaag cacttttgaa acaagcagta catccaagct agaaacttct 4140

ggaaggcatc cagatgcctc tgcaaccgcg actgatgctg tgagttcaga agcccccctc 4200

tcacccacaa tcgaagaaaa ggtcatgttg tgcattcagg aaaatgtgga aaagggccaa 4260

gtgcaaacaa agcccacctc tgtggaagca aagcagaagc ctgggccttc ttttgccagc 4320

tggtttggtt ttcggaagag tagacttcca gctctgagta gcaggaaaat ggacatctcc 4380

aaaaccaaag tagaaaagaa agatgcaaaa gtcttggggt ttggaaacag acaactaaaa 4440

tcagaaagaa aaaaagagaa aaagaagcct gaactacagt gtgagacaga aaatgagctt 4500

atcaaggaca ccaagtcagc agataatcca gatggcggtt tacaaagcaa aaataatcgg 4560

agaacaccac aagacattta caaccaactg aagattgaac caaggaatag acacagccct 4620

gttgcatgtt caacgaaaga caccttcatg acggaactct tgaacagagt tgataagaaa 4680

gcagctccac agacagaaag tggatcaagt aatgcttcct gcaggaatgt gttaaagggc 4740

agttctcagg gctcctgtct catcggcagc tctatcagta ctcaaggaaa ccacaagaaa 4800

aacatgaaaa tcaaagccga tatggaagta ccaaaagact ccctggtaaa agaggcaaat 4860

gaaaacttgc aagaggatga agacgatgca gttgcagatt ctgtatttca gagccacatc 4920

atagaatcca actgccagat gagaacattg gacagtggga tcggaacctt tccactccca 4980

gactcgggaa atcgctcgac aggacgctac ctatgccagc cagactcccc agaggacgct 5040

gagcctctcc tgcctctcca gtcagccctt tctgcagttt cttccatgag agcccaaacc 5100

cttgaacgtg aagtgccttc ctccacagac ggccagcgcc ctgcagatag cgccattgtt 5160

cattccacat ccgaccccat catgaccgcc agagggatga ggcctcttca gagccgcctc 5220

cccaaaccag cttcctcagg aaaagtcagt tcccaaaagc agaatgaagc agagccaagg 5280

cctcagacat gctcatcatt cggatatgct gaagacccaa tggcaagcca gccgcttcca 5340

gactggggga gtgaagttgc tgccaccggg acccaggaca aggcacccag aatgtgtacg 5400

tactctgcca gcggtggcag taatagtgac agtgacctgg actatggaga taatggtttt 5460

ggagctggaa ggggacagtt agtgaaagca ctgaagagcg ctgccccaga aattgagaca 5520

acttga 5526

<210> 3
<211> 1017
<212> DNA
<213> mouse

<400> 3
atgcacgaga agctgataca tgagttggaa gaggaacggc acttgcggct acagagtgag 60

gagcggctgc gggaggtgac cctggagtca gagcgcaaca ggatccagat gctcggcctg 120

cagcagcagt tttccagaat ggaagaaact gtccgaagtc tgttgcagag ccaaggatct 180

ccagagcaga aaaaggaaga acctgccaag atcacagcct accaggaagg actacctgat 240

gaagagagaa aagagcaagc agcactgggt gatctccatg tggtggcagc tgatgaggac 300

tcaagaagcg agcacagtag tgtggaggag ggaaaggaag acagcagact gctcctgaag 360

agactgaagg ccctggaggc ggagaattca gcactggcac tggaaaacga gaatcagaga 420

gaacagtatg agcgatgtct tgatgaggta gccaaccaag ttgttcgagc tctactcact 480

caaaaggatc tgagggagga gtgtgtaaag ttgaaaacaa gagtgtttga cttggagcaa 540

cagaaccgag cactcagcct cctgttccaa cagcgagtca gaccagcttc tgatgtgctt 600

ctccaggggg ccgaggtcaa tgaagatgta caagaagatg aagaagatag agttgctgac 660

actactctcc agagccacat gatagaatcc aactgccaga tgagaacact ggacagcggg 720

attggaacct ttcccctccc agactcagga aaccgctcag tgggacgata catatgccag 780

caggactctc cagaggacac tgatgccctc ctgcctctcc agccagccgc ttccatggca 840

tcctctgcca gagcccaaac ccttgaccgc gaagtgcctt cctccacaga cagccagagc 900

tccccggagt ccatgatcat ccattccata tctgacccca gcatgatggc cagagagatg 960

agagcctcac agagtcacct tcccaagcct acctcctcag gaaccgaaac aagttga 1017

<210> 4
<211> 5334
<212> DNA
<213> mouse

<400> 4
atgcacgaga agctgataca tgagttggaa gaggaacggc acttgcggct acagagtgag 60

gagcggctgc gggaggtgac cctggagtca gagcgcaaca ggatccagat gctcggcctg 120
cagcagcagt tttccagaat ggaagaaact gtccgaagtc tgttgcagag ccaaggatct 180
ccagagcaga aaaaggaaga acctgccaag atcacagcct accaggaagg actacctgat 240
gaagagagaa aagagcaagc agcactgggt gatctccatg tggtggcagc tgatgaggac 300
tcaagaagcg agcacagtag tgtggaggag ggaaaggaag acagcagact gctcctgaag 360
agactgaagg ccctggaggc ggagaattca gcactggcac tggaaaacga gaatcagaga 420
gaacagtatg agcgatgtct tgatgaggta gccaaccaag ttgttcgagc tctactcact 480
caaaaggatc tgagggagga gtgtgtaaag ttgaaaacaa gagtgtttga cttggagcaa 540
cagaaccgag cactcagcct cctgttccaa cagcgagtca gaccagcttc tgatgtgctt 600
ctccagcaat catcgcggat ctgggataaa agactaagta ttgattcttc gctcccaagt 660
ggcttcgcca gccctgcaga tgaactacct ccgacccgta tcaaagaaag ccacattcta 720
gagggactga ggaagctaca gaagcggaaa gtgttccttg aatcaccatc attggtgata 780
agcaaatggg gttataaaga ttgcatgaac tcgaatgaag ggatctattc gccaggaatt 840
aaaaccagca gcctcaagga gtgtccgccc tgccaaccgt ctggcatggg gatcccctgc 900
cagaatctcc atcagacatt catctatgat gcaaattctc atgaggatgc acatgaagaa 960
ctcccatcct cagccttgcc actaacatct ccgagccagg gctacagaat acagggcagt 1020
aaattgacac acagtgtttc agacagtctg tttaactggg agccacatag gaagtacttt 1080
gcagaaggta cagcctcagt atataccaga gaaatgcctg aaaggctagc tcgttgtgct 1140
agccactgcc ccttggagag gaagctatgt cccagtatac acctgccctg ggtgcaaaga 1200
gacagggacc cacacagcca agttcatggc catgtgcctc tgaacctgca gctctcagat 1260

accgatgaca atgagaccct aaacgagcta cacatagaga gcagtgatga gagaagccct 1320

tcagacctgt ccttgactgg ggacaccgac aagtccatgg agaacctaga ggtgggattc 1380

agaaagtctg aaggtggctc tcgggacatg ggagaagaag catgtcccac ccacttagag 1440

actagcccga aggcattcag tttcatcaag caacaaaggg ttgtcaagag gacctcttcg 1500

gaagaatgtg tcacggtcat atttgatgca gaaaatggag agccagtgga attcagctct 1560

caccaggctg gagtcgtcac tgtcaccagg gatgaaattt ccatcagctc agcccttgct 1620

gggccctcag aggaactcac tgaactttca cctcaggaaa ttactcactt ccaggcagga 1680

gctactgcaa gagactgtcc ttttctgaag agacctgaag aggagactgc gaaaaacatt 1740

ccaaaagatg aaaatcatat tctcacagct ccgaatgact tcttcagctc ccagactata 1800

ataactcaga attccccaag accaaagctc gccaaaccca cacagggggt agcctggcag 1860

agtaattcta gacctgttgg aaacatgggt gtctacacaa agcaaggtct gacaaaaatc 1920

ccttccagag gcaagtcttc acctcagaaa tccaaacttg tggagtctat gcccaccatg 1980

ctggtcccct ccagtgccct agtgactcct gaaaagacac cagcatcccc tatggggaaa 2040

ctctcacaat gtagaaaggc agagagccct ggttctctct gtgatgtaca gccagaatca 2100

cacattccaa aacaccctgc ccaggttcct catatcccca aaatttccaa taagaagaac 2160

tgggttcagt gccccaggag tcagacttca gggatccagt cacggcatct catggggaga 2220

agtgacagta gtgaaatgag gtccaatggc tgtgtctctg cgccacagac aagaatgagg 2280

tctccctccc ctccaacccc tccagggaga tcagtgtcac tcctggttag acccagttat 2340

gattacccac cactaccatc acctgccaaa cctgaaactc agggccctgg agacgctgta 2400

agtccagggt tgaaatcccc gctgttgaaa ggatcctctg ctcctcttgt ctcttccagt 2460

ccagtgatgt cagaaataca gaagaaacca tctgtggcct ccagaaagtc tgccttccct 2520

ccagctcttc cctcaaccca agcggtagtg cacaccagat accctgaaca catagcctcc 2580

agtccatttg ctgtgacgac ttcagggccc ccaaaagtct ctccaaaaag aggcatccct 2640

aaacctcccc ctcaccatgc actaggaact gcccacatgg acacagactt acagacaccc 2700

aagacttgtc cttcaagctg tgaactgcta gaggtgacat cttgtaaaag cctgtctcca 2760

gggagaaaaa gacagttgag tgacagcact gtgatgcccc acaggccttc cttctcagga 2820

gtgaatgagt ccttgacacc tcaggttaac agtccatcct cctcttcctc atcctgtaga 2880

aaccaagggt cctcacaagg tggccagaac tctcccgaga aaggacctaa aactcgcctc 2940

ccagtaggac tcaaagttct catgaagtct cctcagttgc tcaagaaaac ttccttggta 3000

ccagggaaac aagaaaagga tagtttaaat gaagcatctc gaagctgtgt agcagcaaac 3060

acgtctaatc ctgaggactc caaagaccct gcaagtctgg agacctcaag agatgagaga 3120

accacagcct tactaggtgt gagaggtccc gacagtctag ctagaggact tgccttagac 3180

tcaacaatgc cagaatcgtt agaatgtagc atctccagag atgatggaag agatggggca 3240

gacagcagag ccataaaaag atccttctcc tccagcaagc cacacctaaa gcctgctcta 3300

ggcatgaatg gagccaaagc tcggagtcag agcttcagca tccacttggg agagaagcct 3360

tccacacccc caacagaagg gccaggcaag attcgaactc agattatcac caacacggct 3420

gaaagaggca actccctcac cagacagaat atctcaatgg aagggtcccc aagcaagacc 3480

ccctctgtct caacatctga taatctgccc aatgctggaa ggcccctggg aaaccccttt 3540

tccaggcaga gttctctctg tagcaccgga agttctagca gccagcacgg aagcccaagc 3600

aagttaccct tgaggatgct cacaaggccg gaaggactcc tcactcctgg agtggaagac 3660

caacggctct ctactcagga agagtgtcct gaaagaggtg tacatgagca atctagcagt 3720

gaccaccata ggcatccatc tcctctttca gactgccctc agttcctgca cgttccaggg 3780

aggatgcagc gcccaagcaa tttggaaaca agtgggatat ctaagctaga aacttctgga 3840

aggcatccag atgcttcttc aactgggact agtgtcgtga gcccagaagc ccccctctca 3900

ccctccatcg aagaaaaggt catgttgtgc attcaggaga atgtggaaaa gggccaagtg 3960

cagacaaagt ctgcctctgt ggaagtgaag cccagggcag ggccttcgtt caccagctgg 4020

tttggttttc gaaagagcag gcttccagct ctcagtagca ggagaatgga agcttccaag 4080

agtaaagtgg aaaagaaaga cacaaaactg aaatctgaga ggaaaaaaga aaagaagaaa 4140

gctgaagtac aatcaaagat agaaaacgaa ctgagtaggg ggaccaagaa ggcagatggt 4200

cagagtccag acgatggtct gcaaagctca gagagtctga aagtatccca agacatttat 4260

aaccaaatga agttggagcc aaggaataga cccagccctg ctgtgtgccc aacaaaagat 4320

gccttcatga cagaacttct aaacagagtt gataagagag cctcggtaca gagagagagt 4380

ggatcaaagg gtgtttcctg caggagcgtg ttagagggca cctcccaggg ctcctgtttc 4440

gccagcggct ctgtgagcac tcaaggaagt caaaagaaaa acatcaaaac caaagtcgat 4500

atggaaaaac caagagaatc cttgggggcc gaggtcaatg aagatgtaca agaagatgaa 4560

gaagatagag ttgctgacac tactctccag agccacatga tagaatccaa ctgccagatg 4620

agaacactgg acagcgggat tggaaccttt cccctcccag actcaggaaa ccgctcagtg 4680

ggacgataca tatgccagca ggactctcca gaggacactg atgccctcct gcctctccag 4740

ccagccgctt ccatggcatc ctctgccaga gcccaaaccc ttgaccgcga agtgccttcc 4800

tccacagaca gccagagctc cccggagtcc atgatcatcc attccatatc tgaccccagc 4860

atgatggcca gagagatgag agcctcacag agtcaccttc ccaagcctac ctcctcagga 4920

aaacctagtt ttgaaaagca aaatggagca gagtcaaagg ctcacactgg cccatcattc 4980

gaatatgctg aggatgcagt ggcaagtgag cccctttcag gatggagggg tgaagatgct 5040

actactgaga cccaggaacc gaaacaagtt gaagaaacaa aagaagatcc tgagaataga 5100

ttatctgaaa tttccctgga gtcattcaat aaatttagca gcaatactgt gattttatta 5160

ggaaaagaga agtctccaag tgaggttgaa ggacaaaagg aagagaaagg caaaaagaca 5220

gaagtgtctt cgagtagctc cgaacggcct ggggtggacc gtgtggagtc tctgagtgac 5280

tctctgtatg acagtttctc ctcttgtgca agtcaagcat ccaacgaggc ctag 5334

<210> 5
<211> 267
<212> DNA
<213> Homo sapiens

<400> 5
gacaaatgcg ttctcgaggt ggagagaaaa cataaggaag ctttggaaga tcttcacatg 60

gtggttgatg aggattcgag gagtgaaagc agcagtacag atgagggaaa agaaaagacc 120

aaattgctat tagagagatt gaaagctcta gaggcagaga attcagcgtt ggctttggag 180

aatgaaaatc aaagggaaca atatgagcga tgtcttgatg aggtagccaa ccaagttgtt 240

caagctctgc ttactcaaaa ggataag 267

<210> 6
<211> 89
<212> PRT
<213> Homo sapiens

<400> 6

Asp Lys Cys Val Leu Glu Val Glu Arg Lys His Lys Glu Ala Leu Glu
1               5                   10                  15

Asp Leu His Met Val Val Asp Glu Asp Ser Arg Ser Glu Ser Ser Ser
            20                  25                  30

Thr Asp Glu Gly Lys Glu Lys Thr Lys Leu Leu Leu Glu Arg Leu Lys
        35                  40                  45

Ala Leu Glu Ala Glu Asn Ser Ala Leu Ala Leu Glu Asn Glu Asn Gln
    50                  55                  60

Arg Glu Gln Tyr Glu Arg Cys Leu Asp Glu Val Ala Asn Gln Val Val
65                  70                  75                  80

Gln Ala Leu Leu Thr Gln Lys Asp Lys
                85

<210> 7
<211> 583
<212> PRT
<213> Homo sapiens

<400> 7

Glu Ala Ala Ala Gly Gly Lys Val Leu Arg Glu Glu Asn Gln Cys Ile
1               5                   10                  15

Ala Pro Val Val Ser Ser Arg Val Ser Pro Gly Thr Arg Pro Thr Ala
            20                  25                  30

Met Gly Ser Phe Ser Ser His Met Thr Glu Phe Pro Arg Lys Arg Lys
        35                  40                  45

Gly Ser Asp Ser Asp Pro Ser Gln Glu Ala His Ser Gln Thr Glu Lys
    50                  55                  60

Arg Arg Arg Asp Lys Met Asn Asn Leu Ile Glu Glu Leu Ser Ala Met
65                  70                  75                  80

Ile Pro Gln Cys Asn Pro Met Ala Arg Lys Leu Asp Lys Leu Thr Val
                85                  90                  95

Leu Arg Met Ala Val Gln His Leu Arg Ser Leu Lys Gly Leu Thr Asn
            100                 105                 110

Ser Tyr Val Gly Ser Asn Tyr Arg Pro Ser Phe Leu Gln Asp Asn Glu
        115                 120                 125

Leu Arg His Leu Ile Leu Lys Thr Ala Glu Gly Phe Leu Phe Val Val
    130                 135                 140

Gly Cys Glu Arg Gly Lys Ile Leu Phe Val Ser Lys Ser Val Ser Lys
145                 150                 155                 160

Ile Leu Asn Tyr Asp Gln Ala Ser Leu Thr Gly Gln Ser Leu Phe Asp
                165                 170                 175

Phe Leu His Pro Lys Asp Val Ala Lys Val Lys Glu Gln Leu Ser Ser
            180                 185                 190

Phe Asp Ile Ser Pro Arg Glu Lys Leu Ile Asp Ala Lys Thr Gly Leu
        195                 200                 205

Gln Val His Ser Asn Leu His Ala Gly Arg Thr Arg Val Tyr Ser Gly
    210                 215                 220

Ser Arg Arg Ser Phe Phe Cys Arg Ile Lys Ser Cys Lys Ile Ser Val
225                 230                 235                 240

Lys Glu Glu His Gly Cys Leu Pro Asn Ser Lys Lys Lys Glu His Arg
                245                 250                 255

Lys Phe Tyr Thr Ile His Cys Thr Gly Tyr Leu Arg Ser Trp Pro Pro
            260                 265                 270

Asn Ile Val Gly Met Glu Glu Glu Arg Asn Ser Lys Lys Asp Asn Ser
        275                 280                 285

Asn Phe Thr Cys Leu Val Ala Ile Gly Arg Leu Gln Pro Tyr Ile Val
    290                 295                 300

Pro Gln Asn Ser Gly Glu Ile Asn Val Lys Pro Thr Glu Phe Ile Thr
305                 310                 315                 320

Arg Phe Ala Val Asn Gly Lys Phe Val Tyr Val Asp Gln Arg Ala Thr
                325                 330                 335

Ala Ile Leu Gly Tyr Leu Pro Gln Glu Leu Leu Gly Thr Ser Cys Tyr
            340                 345                 350

Glu Tyr Phe His Gln Asp Asp His Asn Asn Leu Thr Asp Lys His Lys
        355                 360                 365

Ala Val Leu Gln Ser Lys Glu Lys Ile Leu Thr Asp Ser Tyr Lys Phe
    370                 375                 380

Arg Ala Lys Asp Gly Ser Phe Val Thr Leu Lys Ser Gln Trp Phe Ser
385                 390                 395                 400

Phe Thr Asn Pro Trp Thr Lys Glu Leu Glu Tyr Ile Val Ser Val Asn
                405                 410                 415

Thr Leu Val Leu Gly His Ser Glu Pro Gly Glu Ala Ser Phe Leu Pro
            420                 425                 430

Cys Ser Ser Gln Ser Ser Glu Glu Ser Ser Arg Gln Ser Cys Met Ser
        435                 440                 445

Val Pro Gly Met Ser Thr Gly Thr Val Leu Gly Ala Gly Ser Ile Gly
    450                 455                 460

Thr Asp Ile Ala Asn Glu Ile Leu Asp Leu Gln Arg Leu Gln Ser Ser
465                 470                 475                 480

Ser Tyr Leu Asp Asp Ser Ser Pro Thr Gly Leu Met Lys Asp Thr His
                485                 490                 495

Thr Val Asn Cys Arg Ser Met Ser Asn Lys Glu Leu Phe Pro Pro Ser
            500                 505                 510

Pro Ser Glu Met Gly Glu Leu Glu Ala Thr Arg Gln Asn Gln Ser Thr
        515                 520                 525

Val Ala Val His Ser His Glu Pro Leu Leu Ser Asp Gly Ala Gln Leu
    530                 535                 540

Asp Phe Asp Ala Leu Cys Asp Asn Asp Asp Thr Ala Met Ala Ala Phe
545                 550                 555                 560

Met Asn Tyr Leu Glu Ala Glu Gly Gly Leu Gly Asp Pro Gly Asp Phe
                565                 570                 575

Ser Asp Ile Gln Trp Thr Leu
            580

<210> 8
<211> 21
<212> DNA
<213> Artificial

<220>
<223> primer

<400> 8
gggaagaacc tcaggaaaag g                                                21

<210> 9
<211> 21
<212> DNA
<213> Artificial

<220>
<223> primer

<400> 9
cgatcccact gtccaatgtt c                                                21

<210> 10
<211> 19
<212> DNA
<213> Artificial

<220>
<223> primer

<400> 10
cagccctgtt gcatgttca                                                   19

<210> 11
<211> 20
<212> DNA
<213> Artificial

<220>
<223> primer

<400> 11
gccctgagaa ctgcccttta                                                  20

<210> 12
<211> 20
<212> DNA
<213> Artificial

<220>
<223> primer

<400> 12
gtgagcctgg agaagcatca                                                  20

<210> 13
<211> 21
<212> DNA
<213> Artificial

<220>
<223> primer

<400> 13
ccagcaccaa gtactgttcc a 21


<210> 14
<211> 591
<212> DNA
<213> Homo sapiens

<400> 14
atgggcagca gccatcatca tcatcatcac agcagcggcc tggtgccgcg cggcagccat 60

atggctagca tgactggtgg acagcaaatg ggtcgcggat ccactcagca tccaagcact 120

tttgaaacaa gcagtacatc caagctagaa acttctggaa ggcatccaga tgcctctgca 180

accgcgactg atgctgtgag ttcagaagcc cccctctcac ccacaatcga agaaaaggtc 240

atgttgtgca ttcaggaaaa tgtggaaaag ggccaagtgc aaacaaagcc cacctctgtg 300

gaagcaaagc agaagcctgg gccttctttt gccagctggt ttggttttcg gaagagtaga 360

cttccagctc tgagtagcag gaaaatggac atctccaaaa ccaaagtaga aaagaaagat 420

gcaaaagtct tggggtttgg aaacagacaa ctaaaatcag aaagaaaaaa agagaaaaag 480

aagcctgaac tacagtgtga gacagaaaat gagcttatca aggacaccaa gtcagcagat 540

aatccagata ctcgagcacc accaccacca ccactgagat ccggctgcta a 591

<210> 15
<211> 196
<212> PRT
<213> Homo sapiens

<400> 15

Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Thr Gln His Pro Ser Thr Phe Glu Thr Ser Ser Thr Ser Lys
        35                  40                  45

Leu Glu Thr Ser Gly Arg His Pro Asp Ala Ser Ala Thr Ala Thr Asp
    50                  55                  60

Ala Val Ser Ser Glu Ala Pro Leu Ser Pro Thr Ile Glu Glu Lys Val
65                  70                  75                  80

Met Leu Cys Ile Gln Glu Asn Val Glu Lys Gly Gln Val Gln Thr Lys
                85                  90                  95

Pro Thr Ser Val Glu Ala Lys Gln Lys Pro Gly Pro Ser Phe Ala Ser
            100                 105                 110

Trp Phe Gly Phe Arg Lys Ser Arg Leu Pro Ala Leu Ser Ser Arg Lys
        115                 120                 125

Met Asp Ile Ser Lys Thr Lys Val Glu Lys Lys Asp Ala Lys Val Leu

130                 135                 140

Gly Phe Gly Asn Arg Gln Leu Lys Ser Glu Arg Lys Lys Glu Lys Lys
145                 150                 155                 160

Lys Pro Glu Leu Gln Cys Glu Thr Glu Asn Glu Leu Ile Lys Asp Thr
                165                 170                 175

Lys Ser Ala Asp Asn Pro Asp Thr Arg Ala Pro Pro Pro Pro Pro Leu
            180                 185                 190

Arg Ser Gly Cys
        195

<210>  16
<211>  645
<212>  DNA
<213>  Homo sapiens

<400>  16
atgggcagca gccatcatca tcatcatcac agcagcggcc tggtgccgcg cggcagccat      60

atggctagca tgactggtgg acagcaaatg ggtcgcggat cctttccact cccagactcg     120

ggaaatcgct cgacaggacg ctacctatgc cagccagact ccccagagga cgctgagcct     180

ctcctgcctc tccagtcagc cctttctgca gtttcttcca tgagagccca aacccttgaa     240

cgtgaagtgc cttcctccac agacggccag cgccctgcag atagcgccat tgttcattcc     300

acatccgacc ccatcatgac cgccagaggg atgaggcctc ttcagagccg cctccccaaa     360

ccagcttcct caggaaaagt cagttcccaa aagcagaatg aagcagagcc aaggcctcag     420

acatgctcat cattcggata tgctgaagac ccaatggcaa gccagccgct tccagactgg     480

gggagtgaag ttgctgccac cgggacccag gacaaggcac ccagaatgtg tacgtactct 540

gccagcggtg gcagtaatag tgacagtgac ctggactatg gagataatgg ttttggagct 600

ggaactcgag caccaccacc accaccactg agatccggct gctaa 645

<210> 17
<211> 214
<212> PRT
<213> Homo sapiens

<400> 17

Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Phe Pro Leu Pro Asp Ser Gly Asn Arg Ser Thr Gly Arg Tyr
        35                  40                  45

Leu Cys Gln Pro Asp Ser Pro Glu Asp Ala Glu Pro Leu Leu Pro Leu
    50                  55                  60

Gln Ser Ala Leu Ser Ala Val Ser Ser Met Arg Ala Gln Thr Leu Glu
65                  70                  75                  80

Arg Glu Val Pro Ser Ser Thr Asp Gly Gln Arg Pro Ala Asp Ser Ala
                85                  90                  95

Ile Val His Ser Thr Ser Asp Pro Ile Met Thr Ala Arg Gly Met Arg

            100                 105                 110

Pro Leu Gln Ser Arg Leu Pro Lys Pro Ala Ser Ser Gly Lys Val Ser
        115                 120                 125

Ser Gln Lys Gln Asn Glu Ala Glu Pro Arg Pro Gln Thr Cys Ser Ser
    130                 135                 140

Phe Gly Tyr Ala Glu Asp Pro Met Ala Ser Gln Pro Leu Pro Asp Trp
145                 150                 155                 160

Gly Ser Glu Val Ala Ala Thr Gly Thr Gln Asp Lys Ala Pro Arg Met
                165                 170                 175

Cys Thr Tyr Ser Ala Ser Gly Gly Ser Asn Ser Asp Ser Asp Leu Asp
            180                 185                 190

Tyr Gly Asp Asn Gly Phe Gly Ala Gly Thr Arg Ala Pro Pro Pro Pro
        195                 200                 205

Pro Leu Arg Ser Gly Cys
    210

<210> 18
<211> 861
<212> DNA
<213> mouse

<400> 18
atgggcagca gccatcatca tcatcatcac agcagcggcc tggtgccgcg cggcagccat 60

atggctagca tgactggtgg acagcaaatg ggtcgcggat ccggaaagga agacagcaga 120

ctgctcctga agagactgaa ggccctggag gcggagaatt cagcactggc actggaaaac 180

gagaatcaga gagaacagta tgagcgatgt cttgatgagg tagccaacca agttgttcga 240

gctctactca ctcaaaagga tctgagggag gagtgtgtaa agttgaaaac aagagtgttt 300

gacttggagc aacagaaccg agcactcagc ctcctgttcc aacagcgagt cagaccagct 360

tctgatgtgc ttctccagga acacctccag aatgcaaaat caggaacccc tgccttgaaa 420

cactctggct caggggttgt catccctgat cacctctgtc ctcgcaacag ctgcagcagt 480

ggcagtgagc tatcgctgtc aagcacctgc agcgagtact ccagtggctc ctcctacacg 540

tggcaagaca ggaagctcat tgggaaaagg caatcatcgc ggatctggga taaaagacta 600

agtattgatt cttcgctccc aagtggcttc gccagccctg cagatgaact acctccgacc 660

cgtatcaaag aaagccacat tctagaggga ctgaggaagc tacagaagcg gaaagtgttc 720

cttgaatcac catcattggt gataagcaaa tggggttata aagattgcat gaactcgaat 780

gaagggatct attcgccagg aattaaaacc agcagcctca ctcgagcacc accaccacca 840

ccactgagat ccggctgcta a 861

<210> 19
<211> 286
<212> PRT
<213> mouse

<400> 19

Met Gly Ser Ser His His His His His His Ser Ser Gly Leu Val Pro
1               5                   10                  15

Arg Gly Ser His Met Ala Ser Met Thr Gly Gly Gln Gln Met Gly Arg

            20                  25                  30

Gly Ser Gly Lys Glu Asp Ser Arg Leu Leu Leu Lys Arg Leu Lys Ala
        35                  40                  45

Leu Glu Ala Glu Asn Ser Ala Leu Ala Leu Glu Asn Glu Asn Gln Arg
    50                  55                  60

Glu Gln Tyr Glu Arg Cys Leu Asp Glu Val Ala Asn Gln Val Val Arg
65                  70                  75                  80

Ala Leu Leu Thr Gln Lys Asp Leu Arg Glu Glu Cys Val Lys Leu Lys
                85                  90                  95

Thr Arg Val Phe Asp Leu Glu Gln Gln Asn Arg Ala Leu Ser Leu Leu
            100                 105                 110

Phe Gln Gln Arg Val Arg Pro Ala Ser Asp Val Leu Leu Gln Glu His
        115                 120                 125

Leu Gln Asn Ala Lys Ser Gly Thr Pro Ala Leu Lys His Ser Gly Ser
    130                 135                 140

Gly Val Val Ile Pro Asp His Leu Cys Pro Arg Asn Ser Cys Ser Ser
145                 150                 155                 160

Gly Ser Glu Leu Ser Leu Ser Ser Thr Cys Ser Glu Tyr Ser Ser Gly
                165                 170                 175

Ser Ser Tyr Thr Trp Gln Asp Arg Lys Leu Ile Gly Lys Arg Gln Ser

            180                 185                 190

Ser Arg Ile Trp Asp Lys Arg Leu Ser Ile Asp Ser Ser Leu Pro Ser
        195                 200                 205

Gly Phe Ala Ser Pro Ala Asp Glu Leu Pro Pro Thr Arg Ile Lys Glu
    210                 215                 220

Ser His Ile Leu Glu Gly Leu Arg Lys Leu Gln Lys Arg Lys Val Phe
225                 230                 235                 240

Leu Glu Ser Pro Ser Leu Val Ile Ser Lys Trp Gly Tyr Lys Asp Cys
                245                 250                 255

Met Asn Ser Asn Glu Gly Ile Tyr Ser Pro Gly Ile Lys Thr Ser Ser
            260                 265                 270

Leu Thr Arg Ala Pro Pro Pro Pro Pro Leu Arg Ser Gly Cys
        275                 280                 285

<210> 20
<211> 669
<212> DNA
<213> mouse

<400> 20
atgggcagca gccatcatca tcatcatcac agcagcggcc tggtgccgcg cggcagccat 60

atggctagca tgactggtgg acagcaaatg ggtcgcggat ccggaaagga agacagcaga 120

ctgctcctga agagactgaa ggccctggag gcggagaatt cagcactggc actggaaaac 180

gagaatcaga gagaacagta tgagcgatgt cttgatgagg tagccaacca agttgttcga 240

gctctactca ctcaaaagga tctgagggag gagtgtgtaa agttgaaaac aagagtgttt 300

gacttggagc aacagaaccg agcactcagc ctcctgttcc aacagcgagt cagaccagct 360

tctgatgtgc ttctccagca atcatcgcgg atctgggata aaagactaag tattgattct 420

tcgctcccaa gtggcttcgc cagccctgca gatgaactac ctccgacccg tatcaaagaa 480

agccacattc tagagggact gaggaagcta cagaagcgga aagtgttcct tgaatcacca 540

tcattggtga taagcaaatg gggttataaa gattgcatga actcgaatga agggatctat 600

tcgccaggaa ttaaaaccag cagcctcact cgagcaccac caccaccacc actgagatcc 660

ggctgctaa 669

<210> 21
<211> 222
<212> PRT
<213> mouse

<400> 21

Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Gly Lys Glu Asp Ser Arg Leu Leu Leu Lys Arg Leu Lys Ala
        35                  40                  45

Leu Glu Ala Glu Asn Ser Ala Leu Ala Leu Glu Asn Glu Asn Gln Arg
    50                  55                  60

Glu Gln Tyr Glu Arg Cys Leu Asp Glu Val Ala Asn Gln Val Val Arg
65                  70                  75                  80

Ala Leu Leu Thr Gln Lys Asp Leu Arg Glu Glu Cys Val Lys Leu Lys
                85                  90                  95

Thr Arg Val Phe Asp Leu Glu Gln Gln Asn Arg Ala Leu Ser Leu Leu
            100                 105                 110

Phe Gln Gln Arg Val Arg Pro Ala Ser Asp Val Leu Leu Gln Gln Ser
        115                 120                 125

Ser Arg Ile Trp Asp Lys Arg Leu Ser Ile Asp Ser Ser Leu Pro Ser
    130                 135                 140

Gly Phe Ala Ser Pro Ala Asp Glu Leu Pro Pro Thr Arg Ile Lys Glu
145                 150                 155                 160

Ser His Ile Leu Glu Gly Leu Arg Lys Leu Gln Lys Arg Lys Val Phe
                165                 170                 175

Leu Glu Ser Pro Ser Leu Val Ile Ser Lys Trp Gly Tyr Lys Asp Cys
            180                 185                 190

Met Asn Ser Asn Glu Gly Ile Tyr Ser Pro Gly Ile Lys Thr Ser Ser
        195                 200                 205

Leu Thr Arg Ala Pro Pro Pro Pro Pro Leu Arg Ser Gly Cys
    210                 215                 220

<210> 22
<211> 633
<212> DNA
<213> Homo sapiens

<400> 22
atgggcagca gccatcatca tcatcatcac agcagcggcc tggtgccgcg cggcagccat 60

atggctagca tgactggtgg acagcaaatg ggtcgcggat ccctctcacc ctccatcgaa 120

gaaaaggtca tgttgtgcat tcaggagaat gtggaaaagg gccaagtgca gacaaagtct 180

gcctctgtgg aagtgaagcc cagggcaggg ccttcgttca ccagctggtt tggttttcga 240

aagagcaggc ttccagctct cagtagcagg agaatggaag cttccaagag taaagtggaa 300

aagaaagaca caaaactgaa atctgagagg aaaaaagaaa agaagaaagc tgaagtacaa 360

tcaaagatag aaaacgaact gagtaggggg accaagaagg cagatggtca gagtccagac 420

gatggtctgc aaagctcaga gagtctgaaa gtatcccaag acatttataa ccaaatgaag 480

ttggagccaa ggaatagacc cagccctgct gtgtgcccaa caaaagatgc cttcatgaca 540

gaacttctaa acagagttga taagagagcc tcggtacaga gagagagtgg aactcgagca 600

ccaccaccac caccactgag atccggctgc taa 633

<210> 23
<211> 210
<212> PRT
<213> Homo sapiens

<400> 23

Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Leu Ser Pro Ser Ile Glu Glu Lys Val Met Leu Cys Ile Gln
        35                  40                  45

Glu Asn Val Glu Lys Gly Gln Val Gln Thr Lys Ser Ala Ser Val Glu
    50                  55                  60

Val Lys Pro Arg Ala Gly Pro Ser Phe Thr Ser Trp Phe Gly Phe Arg
65                  70                  75                  80

Lys Ser Arg Leu Pro Ala Leu Ser Ser Arg Arg Met Glu Ala Ser Lys
                85                  90                  95

Ser Lys Val Glu Lys Lys Asp Thr Lys Leu Lys Ser Glu Arg Lys Lys
            100                 105                 110

Glu Lys Lys Lys Ala Glu Val Gln Ser Lys Ile Glu Asn Glu Leu Ser
        115                 120                 125

Arg Gly Thr Lys Lys Ala Asp Gly Gln Ser Pro Asp Asp Gly Leu Gln
    130                 135                 140

Ser Ser Glu Ser Leu Lys Val Ser Gln Asp Ile Tyr Asn Gln Met Lys
145                 150                 155                 160

Leu Glu Pro Arg Asn Arg Pro Ser Pro Ala Val Cys Pro Thr Lys Asp
                165                 170                 175

Ala Phe Met Thr Glu Leu Leu Asn Arg Val Asp Lys Arg Ala Ser Val
            180                 185                 190

Gln Arg Glu Ser Gly Thr Arg Ala Pro Pro Pro Pro Pro Leu Arg Ser
        195                 200                 205

Gly Cys
    210

<210> 24
<211> 627
<212> DNA
<213> Homo sapiens

<400> 24
atgggcagca gccatcatca tcatcatcac agcagcggcc tggtgccgcg cggcagccat      60

atggctagca tgactggtgg acagcaaatg ggtcgcggat ccccttcctc cacagacagc     120

cagagctccc cggagtccat gatcatccat tccatatctg accccagcat gatggccaga     180

gagatgagag cctcacagag tcaccttccc aagcctacct cctcaggaaa acctagtttt     240

gaaaagcaaa atggagcaga gtcaaaggct cacactggcc catcattcga atatgctgag     300

gatgcagtgg caagtgagcc cctttcagga tggaggggtg aagatgctac tactgagacc     360

caggaaccga aacaagttga agaaacaaaa gaagatcctg agaatagatt atctgaaatt     420

tccctggagt cattcaataa atttagcagc aatactgtga ttttattagg aaaagagaag     480

tctccaagtg aggttgaagg acaaaaggaa gagaaaggca aaaagacaga agtgtcttcg     540

agtagctccg aacggcctgg ggtggaccgc gtggagtctc tgagtactcg agcaccacca     600

ccaccaccac tgagatccgg ctgctaa                                         627

<210> 25
<211> 208
<212> PRT
<213> Homo sapiens

<400> 25

Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Pro Ser Ser Thr Asp Ser Gln Ser Ser Pro Glu Ser Met Ile
        35                  40                  45

Ile His Ser Ile Ser Asp Pro Ser Met Met Ala Arg Glu Met Arg Ala
    50                  55                  60

Ser Gln Ser His Leu Pro Lys Pro Thr Ser Ser Gly Lys Pro Ser Phe
65                  70                  75                  80

Glu Lys Gln Asn Gly Ala Glu Ser Lys Ala His Thr Gly Pro Ser Phe
                85                  90                  95

Glu Tyr Ala Glu Asp Ala Val Ala Ser Glu Pro Leu Ser Gly Trp Arg
            100                 105                 110

Gly Glu Asp Ala Thr Thr Glu Thr Gln Glu Pro Lys Gln Val Glu Glu
        115                 120                 125

Thr Lys Glu Asp Pro Glu Asn Arg Leu Ser Glu Ile Ser Leu Glu Ser
    130                 135                 140

Phe Asn Lys Phe Ser Ser Asn Thr Val Ile Leu Leu Gly Lys Glu Lys
145                 150                 155                 160

Ser Pro Ser Glu Val Glu Gly Gln Lys Glu Glu Lys Gly Lys Lys Thr
                165                 170                 175

Glu Val Ser Ser Ser Ser Ser Glu Arg Pro Gly Val Asp Arg Val Glu
            180                 185                 190

Ser Leu Ser Thr Arg Ala Pro Pro Pro Pro Pro Leu Arg Ser Gly Cys
        195                 200                 205

<210>  26
<211>  19
<212>  DNA
<213>  Artificial

<220>
<223>  overhang siRNA

<400>  26
agaccaaatt gctattaga                                                    19

<210>  27
<211>  19
<212>  DNA
<213>  Artificial

<220>
<223>  overhang siRNA

<400> 27
ccaagttgtt caagctctg 19

<210> 28
<211> 19
<212> DNA
<213> Artificial

<220>
<223> overhang siRNA

<400> 28
gctctgctta ctcaaaagg 19

<210> 29
<211> 19
<212> DNA
<213> Artificial

<220>
<223> overhang siRNA

<400> 29
ccagagtact gttgctgtc 19